# EUROPEAN PATENT APPLICATION

(11) **EP 4 431 590 A1**
(43) Date of publication of application: **18.09.2024**
(21) Application number: 22892104.5
(22) Date of filing: 11.11.2022
(51) Int. Cl.: C12M 1/00, C12M 1/34, G01N 35/00

(54) **INTELLIGENT GENE SYNTHESIS AND MONOCLONAL SELECTION SYSTEM**

(30) Priority: 13.11.2021 CN 202111343492
(71) Applicant: Innovel Intelligent Technology Co., Ltd., Suzhou, Jiangsu 215000 (CN); WuXi Biologics Ireland Limited, Dundalk, Co Louth A91 X56F (IE)
(72) Inventor: CHEN, Hao, Suzhou, Jiangsu 215000 (CN); YANG, Kailin, Suzhou, Jiangsu 215000 (CN); ZHI, Yan, Shanghai 200131 (CN); CHEN, Xiaoyue, Shanghai 200131 (CN)
(74) Representative: Casci, Tamara
(86) International application number: PCT/CN2022/131430
(87) International publication number: WO 2023/083311

(57) **Abstract**

An intelligent system for gene synthesis and monoclonal picking, comprising: a core operation module for performing operations associated with gene synthesis and monoclonal picking; and a control system for controlling at least a part of operations of corresponding compartments of the core operation module. The core operation module at least includes: a liquid operation compartment for performing operations of mixing gene fragments and mixing synthetizing reagents with biological samples in the process of gene synthesis; a purification compartment for performing purification operations on nucleic acid products created after the mixing; an amplification compartment for performing amplification operations and error correction operations on fragments of the nucleic acid products and performing culturing and amplification operations on vectors; and a monoclonal picking compartment for performing monoclonal picking operations on purified and cultured nucleic acid products.

## Description

### TECHNICAL FIELD

The present disclosure generally relates to the technical field of biological drugs. More particularly, the present disclosure relates to an intelligent system for gene synthesis and monoclonal picking.

### BACKGROUND ART

Biological drugs are in a period of rapid development. Gene synthesis technology, as an important means for research, development and production of the biological drugs, has been widely applied. The whole process of the gene synthesis technology from primer synthesis to colony identification includes many complicated process steps. A large number of pipetting operations or the like need to be performed in the stage of gene synthesis and amplification. However, the conventional manual production mode or semi-automatic production mode is hard to meet the industrial large-scale production and the related delivery speed of the gene-synthesized drugs. In addition, with respect to the manual or the semi-automatic production mode, it is extremely difficult to increase the throughput due to some restrictions in the aspects of, for example, personnel's technical level and interaction of multiple devices, resulting in low production efficiency, high cost and low quality stability. In the conventional manual operation mode, it takes dozens of people to complete the whole process operation to reach relatively large-scale throughput. Therefore, the conventional manual operation mode requires a huge personnel team and a large venue, and requires, on the front line, highly skilled personnel who have been specially trained, resulting in high human resource costs to research institutions or enterprises. In addition, operation performed by different personnel will bring about some problems related to process stability, reproducibility and the like. Moreover, large-scale manual operation tends to produce confusion errors between samples.

The genes, after having been synthetized, need to be transferred into vector cells/bacteria to be cultured. However, the conventional culturing system cannot be docked with automatic systems (such as robots with high degrees of freedom), so it is unable to realize arrayed culture, and accordingly makes it difficult to realize large-scale experimentation and production. After the vector cells/bacteria have been cultured, monoclonal screening is required to pick out the cells/bacteria that are suitable for continuous amplification for subsequent processes. In order to pick out suitable target cells/bacteria, tens of thousands of cells/bacteria need to be picked every day. However, in the case of manual operation, a single person can only pick hundreds of cells/bacteria every day, which is difficult to meet the demand of high-speed and large-scale research, development and production.

Furthermore, in the conventional operation process, a large number of data need to be collected and analyzed for sample analysis and screening. The conventional manual operation mode tends to cause problems such as incomplete data recording, data loss, data conflict, the need for long-term data saving, and so on.

### CONTENT OF THE INVENTION

One of objects of the present disclosure is to solve one or more of the above problems and realize other additional advantages.

The present disclosure is intended to provide an intelligent system for large-scale gene synthesis and monoclonal picking, so as to achieve the purpose of high-volume production in the upstream process of research and development of macromolecular drugs, and to substantially realize high-throughput screening of the drugs. In addition, it is also expected to realize the online collection of enormous data while effectively replacing large-scale manual operation with intelligent or automatic large-scale operation, to thereby effectively improve the comprehensive efficiency of data collection, sorting, analysis and mining in the research and development process of the biological drugs.

In an aspect of the present disclosure, an intelligent system for gene synthesis and monoclonal picking is provided, comprising: a core operation module for performing operations associated with gene synthesis and monoclonal picking; and a control system for controlling at least a part of operations of corresponding compartments of the core operation module. The core operation module at least includes: a liquid operation compartment for performing operations of mixing gene fragments and operations of mixing synthetizing reagents with biological samples in the process of gene synthesis; a purification compartment for performing purification operations on nucleic acid products created after the mixing; an amplification compartment for performing amplification operations and error correction operations on fragments of the nucleic acid products and performing culturing and amplification operations on vectors; and a monoclonal picking compartment for performing monoclonal picking operations on purified and cultured nucleic acid products.

According to an embodiment of the present disclosure, the core operation module further comprises a loading and unloading compartment for replenishing various materials that are required in the process of gene synthesis and monoclonal picking.

According to an embodiment of the present disclosure, the core operation module further comprises a transfer compartment for transferring biological samples and/or materials among at least a part of the liquid operation compartment, the purification compartment, the amplification compartment, the monoclonal picking compartment and the loading and unloading compartment.

According to an embodiment of the present disclosure, the transfer compartment includes a transfer robot.

According to an embodiment of the present disclosure, the transfer compartment is arranged at a middle position of the core operation module, while the liquid operation compartment, the purification compartment, the amplification compartment, the monoclonal picking compartment and the loading and unloading compartment are divided into two groups and arranged on both sides of the transfer compartment.

According to an embodiment of the present disclosure, the transfer compartment is arranged at a central position of the core operation module, while the liquid operation compartment, the purification compartment, the amplification compartment, the monoclonal picking compartment and the loading and unloading compartment are arranged around the transfer compartment along a circumference.

According to an embodiment of the present disclosure, at least a part of the liquid operation compartment, the purification compartment, the transfer compartment, the amplification compartment, the monoclonal picking compartment and the loading and unloading compartment are arranged in a compartment body capable of being closed tightly, the compartment body including a laminar flow unit to put corresponding compartments in a controlled laminar flow environment.

According to an embodiment of the present disclosure, the laminar flow unit comprises a laminar air-supply unit including an air-supply fan or at least one filtering unit, and a laminar air-discharge unit including an air-discharge fan and a reverse filtering unit.

According to an embodiment of the present disclosure, an ultraviolet irradiation unit is provided in the compartment body.

According to an embodiment of the present disclosure, the liquid operation compartment comprises at least one pipetting unit, the pipetting unit is mounted on a linear motor gantry main frame structure so as enable the pipetting unit to be movable along an X-axis, and each pipetting unit comprises one or more pipetting heads each of which is independently movable along a Y-axis and a Z-axis in the pipetting unit.

According to an embodiment of the present disclosure, each pipetting unit includes one or more movers mounted along a direction of the Y-axis to enable each pipetting head in each pipetting unit to be movable along the Y-axis under driving of the movers, and each pipetting unit further comprises a Z-axis driving mechanism to drive each pipetting head in each pipetting unit to move along the Z-axis.

According to an embodiment of the present disclosure, the liquid operation compartment further comprises one or more of a liquid pumping unit, a pump set array, a bottom-compartment liquid storage unit, a consumable stage sliding table array, an oscillation unit, a consumable carrier and an operation stage.

According to an embodiment of the present disclosure, the liquid operation compartment includes two or more pipetting units, which are configured to be able to perform operations independently of each other as well as to perform operations in parallel.

According to an embodiment of the present disclosure, the purification compartment comprises at least one purification pipetting unit, the at least one purification pipetting unit is mounted on a linear motor gantry main frame structure, so as to enable the at least one purification pipetting unit to be movable along an X-axis, and each purification pipetting unit comprises one or more purification pipetting heads each of which is independently movable along a Y-axis and a Z-axis in the purification pipetting unit.

According to an embodiment of the present disclosure, the purification compartment further comprises one or more of a liquid pumping unit, a consumable stage sliding table array, a bottom-compartment liquid storage unit, an oscillation unit, a consumable carrier, an operation stage and a temperature-controlled storage device.

According to an embodiment of the present disclosure, the monoclonal picking compartment comprises a monoclonal picking unit for picking monoclonal nucleic acid products from an orifice plate.

According to an embodiment of the present disclosure, the monoclonal picking unit comprises a multi-channel pick head assembly formed by integrating a plurality of pick head elements.

According to an embodiment of the present disclosure, the multi-channel pick head assembly is connected to an integrated gas path on-off valve group, which is capable of controlling each pick head element separately to enable each pick head element to independently move and to independently fulfill operations of picking the monoclonal nucleic acid products.

According to an embodiment of the present disclosure, the monoclonal picking unit is mounted on an X-Y linear motor gantry main frame structure, so as to enable the monoclonal picking unit to be movable along an X-axis and a Y-axis, and the monoclonal picking unit includes a Z-axis motor, so as to enable the monoclonal picking unit to be movable along a Z-axis.

According to an embodiment of the present disclosure, the monoclonal picking unit further comprises an image capturing assembly for identifying and localizing target nucleic acid products in the monoclonal picking process.

According to an embodiment of the present disclosure, the image capturing assembly includes: a camera for real-time determining positions of nucleic acid products to be picked; and an optical distance sensor for calibrating a vertical distance between tip ends of the pick head elements in the multi-channel pick head assembly and a surface of the orifice plate bearing the nucleic acid products to be picked, and for evaluating a surface flatness of the orifice plate.

According to an embodiment of the present disclosure, the monoclonal picking unit further comprises a bracket assembly including vertical support plates and a plurality of horizontal support plates extending perpendicular to the vertical support plates, and each horizontal support plate is provided with holes through which the plurality of pick head elements extend, so as to ensure accurate localization of the plurality of pick head elements.

According to an embodiment of the present disclosure, one of the plurality of horizontal support plates is also provided with a guiding and positioning mechanism for the plurality of pick head elements, so as to localize and guide vertical movement of the plurality of pick head elements in the multi-channel pick head assembly.

According to an embodiment of the present disclosure, an upper surface of a bottom horizontal support plate of the plurality of horizontal support plates is configured to function as a limiting surface for each pick head element in the multi-channel pick head assembly, in order to ensure that the tip ends of the plurality of pick head elements are at a same height.

According to an embodiment of the present disclosure, the optical distance sensor and/or the camera are also arranged on the upper surface of the bottom horizontal support plate.

According to an embodiment of the present disclosure, each pick head element includes a first section and a second section, both of which are configured to be in floating connection.

According to an embodiment of the present disclosure, each pick head element includes a floating connection mechanism which includes a sleeve element and a floating element, the floating element is configured to be mountable in the sleeve element and capable of floating a distance within the sleeve element.

According to an embodiment of the present disclosure, an upper end of the first section of each pick head element is connected with a drive control valve to drive vertical movement of the pick head element by the drive control valve, and a lower end of the first section of each pick head element is secured within the sleeve element of the floating connection mechanism.

According to an embodiment of the present disclosure, the lower end of the first section of each pick head element includes a threaded joint for threadedly connecting the lower end of the first section to the sleeve element of the floating connection mechanism.

According to an embodiment of the present disclosure, each pick head element further includes a guide element.

According to an embodiment of the present disclosure, each pick head element further comprises a guide element arranged on an outer periphery of the second section of the pick head element.

According to an embodiment of the present disclosure, the guide element is configured as a guide bearing.

According to an embodiment of the present disclosure, each pick head element is made of SUS316 stainless steel material.

According to an embodiment of the present disclosure, the monoclonal picking compartment further comprises one or more of an oscillation-coating unit, a multi-channel pipetting unit, a liquid pumping unit, a bottom-compartment liquid storage unit, a consumable stage sliding table array, an operation stage, and a temperature-controlled storage device.

According to an embodiment of the present disclosure, the monoclonal picking compartment further comprises an oscillation-coating unit for realizing uniform distribution of flora on the orifice plate, and the oscillation-coating unit comprises a clamping mechanism and a cam oscillating mechanism, the orifice plate capable of being clamped in the clamping mechanism and oscillated together with the clamping mechanism under the drive of the cam oscillating mechanism.

According to an embodiment of the present disclosure, the monoclonal picking compartment further comprises a pick head cleaning assembly including at least one pick head cleaning element, for cleaning the multi-channel pick head assembly of the monoclonal picking unit.

According to an embodiment of the present disclosure, the pick head cleaning assembly further comprises a pick head drying unit including a heating unit, for drying the pick head elements in the multi-channel pick head assembly of the monoclonal picking unit.

According to an embodiment of the present disclosure, the pick head drying unit includes an air-supply duct and an air-discharge duct, for cooling the pick head elements in the multi-channel pick head assembly of the monoclonal picking unit that have been dried.

According to an embodiment of the present disclosure, the control system includes a control computer, an I/O data collecting module, and a drive module.

According to an embodiment of the present disclosure, the control system comprises a software system, which includes one or more of a workflow editing unit, a device monitoring and operating unit, a device debugging unit, a data storage unit, a log prompting unit, and a parameter setting unit.

According to an embodiment of the present disclosure, the intelligent system for gene synthesis and monoclonal picking further comprises at least one of the following modules: a sequencer module for detecting, confirming and screening synthesized gene fragments in the process of gene synthesis and monoclonal picking; and a material storage module for storing various materials required in the process of gene synthesis and monoclonal picking.

The additional and/or other aspects and advantages of the present disclosure will be set forth in the following description, or are obvious from the description or can be learned through the practice of the present disclosure. The various technical features of the present disclosure can be combined arbitrarily as long as they do not contradict each other.

### BRIEF DESCRIPTION OF THE DRAWINGS

With reference to the following detailed description of the specific embodiments of the present disclosure in combination with the accompanying drawings, the above-mentioned features and advantages and other features and advantages of the present disclosure as well as their implementing means will become more apparent. In the figures:
Fig. 1 is a schematic layout view of an intelligent system for gene synthesis and monoclonal picking according to an embodiment of the present disclosure;
Fig. 2 is a schematic layout view of a core operation system according to an embodiment of the present disclosure;
Fig. 3 shows specific structures of the core operation system according to an embodiment of the present disclosure;
Figs. 4a-4c show specific structures of the core operation system according to another embodiment of the present disclosure;
Fig. 5 is a schematic view of a laminar flow unit according to an embodiment of the present disclosure;
Figs. 6a-6b show an operation flow of the system for gene synthesis and monoclonal picking according to different embodiments of the present disclosure;
Figs. 7-10 show specific structures of a liquid operation compartment according to an embodiment of the present disclosure;
Fig. 11 shows a linear motor gantry main frame structure according to an embodiment of the present disclosure;
Fig. 12 shows an operation flow of mixing gene fragments in the liquid operation compartment according to an embodiment of the present disclosure;
Fig. 13 shows specific structures of a purification compartment according to an embodiment of the present disclosure;
Fig. 14 shows an operation flow of performing purification operation in the purification compartment according to an embodiment of the present disclosure;
Figs. 15-16 show specific structures of a monoclonal picking compartment according to an embodiment of the present disclosure;
Figs. 17a-17d show specific structures of a monoclonal picking unit according to an embodiment of the present disclosure;
Figs. 18a-18b show specific structures of a pick head element of the monoclonal picking unit according to an embodiment of the present disclosure;
Fig. 19 shows an operation flow of performing monoclonal picking with the monoclonal picking unit according to an embodiment of the present disclosure;
Fig. 20 shows specific structures of an oscillation-coating assembly according to an embodiment of the present disclosure;
Fig. 21 shows an operation flow of coating flora on an orifice plate with the oscillation-coating assembly according to an embodiment of the present disclosure;
Fig. 22 shows specific structures of a pick head cleaning assembly according to an embodiment of the present disclosure;
Fig. 23 shows specific structures of a pick head drying element of the pick head cleaning assembly according to an embodiment of the present disclosure;
Fig. 24 shows an operation flow of cleaning the pick head element with the pick head cleaning assembly according to an embodiment of the present disclosure;
Fig. 25 shows the basic architecture of a control system according to an embodiment of the present disclosure;
Figs. 26 and 27 show the basic architecture of a software system according to an embodiment of the present disclosure;
Fig. 28 shows another layout of the core operation system according to an embodiment of the present disclosure.

In the drawings, respective reference signs indicate respective components. The examples described herein are used to illustrate exemplary aspects of the present disclosure, and these examples should not be construed as limiting the scope of the present disclosure in any way.

### DATAILED EMBODIMENTS

The present disclosure will be described below with reference to the drawings, in which several embodiments of the present disclosure are shown. It should be understood, however, that the present disclosure may be implemented in many different ways, and is not limited to the example embodiments described below. In fact, the embodiments described hereinafter are intended to make a more complete disclosure of the present disclosure and to adequately explain the scope of the disclosure to a person skilled in the art. It should also be understood that, the embodiments disclosed herein can be combined in various ways to provide many additional embodiments.

For the purpose of description, the terms "upper", "lower", "left", "right", "vertical", "horizontal", "top", "bottom", "transverse", "perpendicular" and their derivatives are all related to the orientation in the drawings of the present disclosure. However, it should be understood that the present disclosure may adopt various alternative modifications, unless otherwise clearly indicated. For example, when the apparatus in the drawings is turned over, the features previously described as being "below" other features may be described to be "above" other features at this time. The apparatus may also be otherwise oriented (rotated 90 degrees or at other orientations) and the relative spatial relationships will be correspondingly altered.

The singular forms "a/an" and "the" as used in the specification, unless clearly indicated, all contain the plural forms. The words "comprising", "containing" and "including" used in the specification indicate the presence of the claimed features, but do not preclude the presence of one or more additional features. The wording "and/or" as used in the specification includes any and all combinations of one or more of the relevant items listed.

In the specification, when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being "directly on", "directly attached" to, "directly connected" to, "directly coupled" with or "directly contacting" another element, there are no intervening elements present. In the specification, references to a feature that is disposed "adjacent" another feature may have portions that overlap, overlie or underlie the adjacent feature.

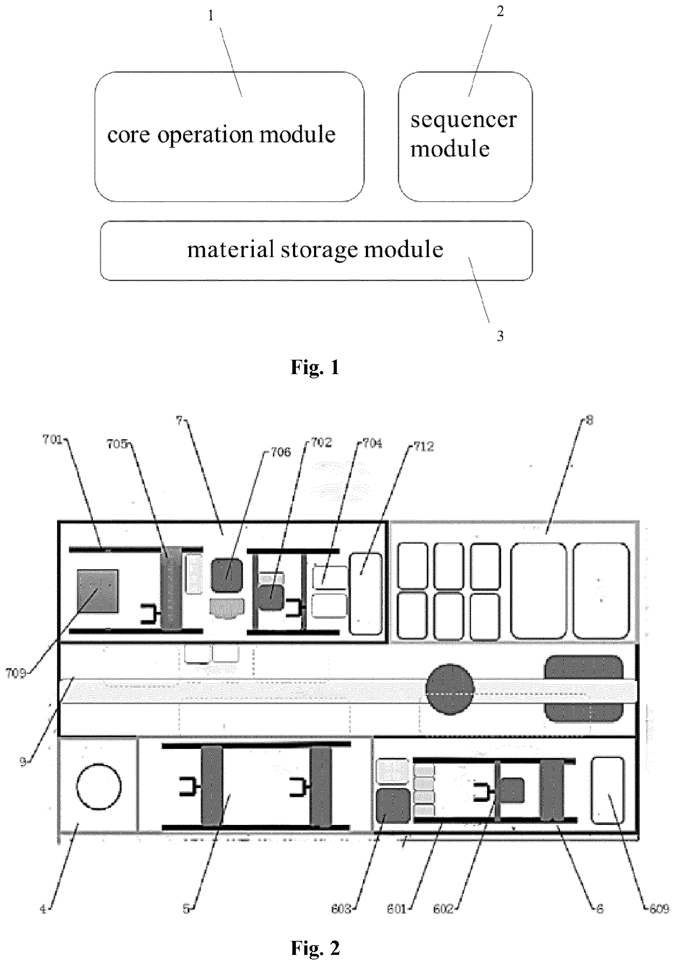

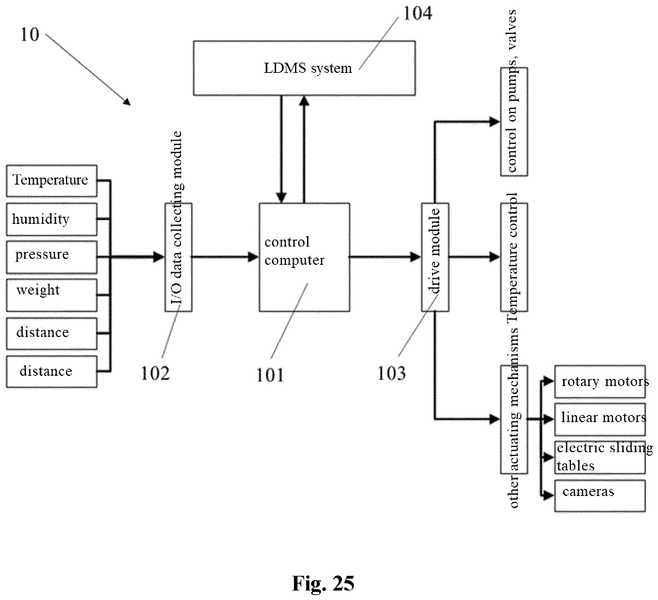

The present disclosure relates to an intelligent system for gene synthesis and monoclonal picking. Referring to Figs. 1 and 25, in an embodiment according to the present disclosure, the intelligent system for gene synthesis and monoclonal picking may include a core operation module 1, a control system 10, and optionally a sequencer module 2 and/or a material storage module 3. In the embodiment according to the present disclosure, the core operation module 1 is used to perform operations associated with gene synthesis and monoclonal picking, for example, core operations such as primer or gene synthesis, PCR amplification, vector amplification, product purification, monoclonal picking and the like. The sequencer module 2 is used to detect, confirm and screen synthesized gene fragments in the process of gene synthesis and monoclonal picking, and the like. The sequencer module 2 may include various required sequencers. The material storage module 3 is used to store various materials such as various consumables, reagents and the like that are required in the process of gene synthesis and monoclonal picking, so as to meet the demand for high-throughput continuous production. The control system 10 is used to control at least a part of operations of corresponding modules of the intelligent system for gene synthesis and monoclonal picking according to the present disclosure (for example, at least a part of operations of the core operation module 1), so as to at least partially or completely realize the automation and intelligentization of the intelligent system for gene synthesis and monoclonal picking according to the present disclosure.

The core operation module 1 may include at least a part or all of a loading and unloading compartment 4, a liquid operation compartment 5, a purification compartment 6, a monoclonal picking compartment 7, an amplification compartment 8, and a transfer compartment 9. The loading and unloading compartment 4 is used to replenish various materials (such as samples for synthesis, synthetizing reagents, consumables in operations and so on) that are required in the process of gene synthesis and monoclonal picking, and to deliver products out after operation. The loading and unloading compartment 4 may include loading and unloading drums. The liquid operation compartment 5 is used to perform operations such as mixing gene fragments, mixing the synthetizing reagents with the samples for synthesis, and the like in the process of gene synthesis. The purification compartment 6 is used to purify nucleic acid products created after the mixing. The monoclonal picking compartment 7 is used to coat the purified nucleic acid products and to perform monoclonal picking on the cultured flora. The amplification compartment 8 is used to perform amplification operations and error correction operations on fragments of the nucleic acid products and to perform culturing and amplification operations on vector bacteria. The transfer compartment 9 is used to transfer the samples and/or materials among the respective compartments. The transfer compartment 9 may include a transfer robot. The transfer robot can carry out various transfer operations under the control of the control system 10. In an embodiment according to the present disclosure, some operations such as concentration detection and centrifugal enrichment may also be performed on the nucleic acid products in the transfer compartment 9.

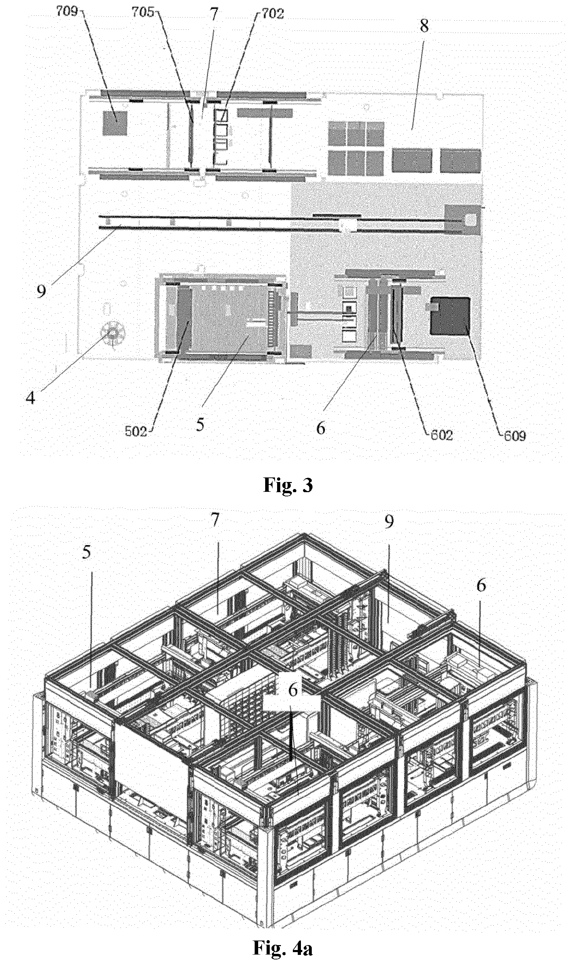

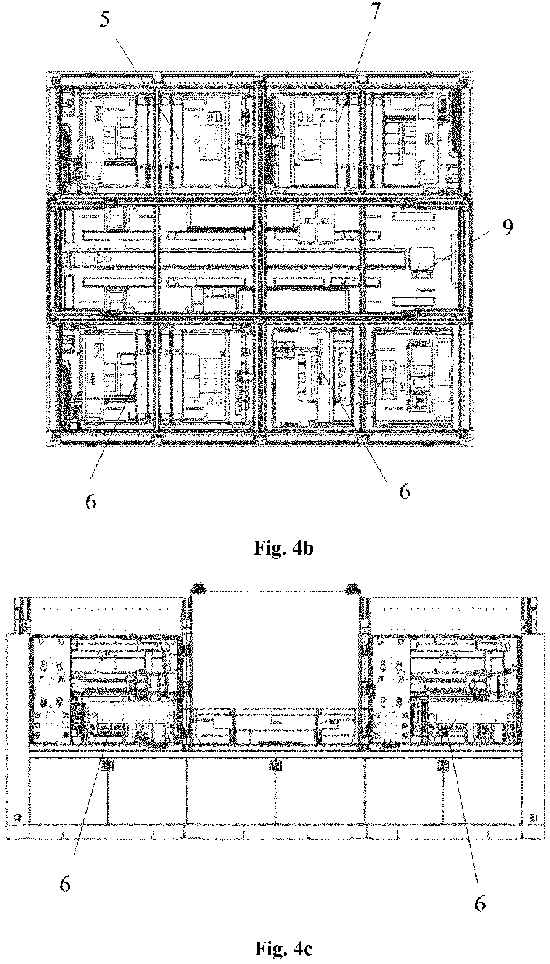

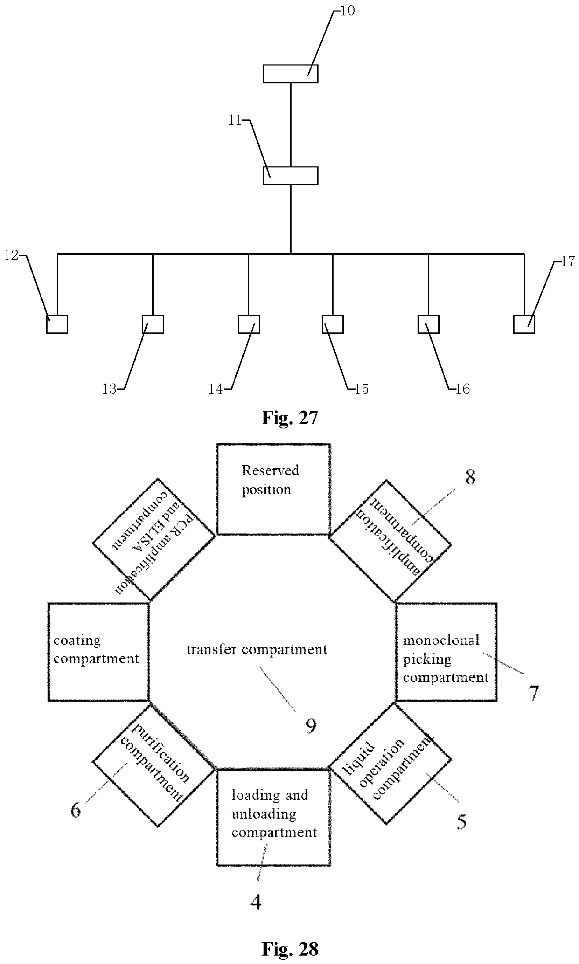

In embodiments according to the present disclosure, a different number of the loading and unloading compartment 4, the liquid operation compartment 5, the purification compartment 6, the monoclonal picking compartment 7, the amplification compartment 8 and the transfer compartment 9 may be provided in the core operation module 1 according to different process requirements, and these compartments may be arranged in any suitable ways in the core operation module 1. For example, referring to Figs. 2 and 3, in an embodiment according to the present disclosure, the transfer compartment 9 may be arranged at a middle position of the core operation module 1, the loading and unloading compartment 4, the liquid operation compartment 5 and the purification compartment 6 may be arranged at a first side of the transfer compartment 9, and the monoclonal picking compartment 7 and the amplification compartment 8 may be arranged at a second side opposing the first side of the transfer compartment 9. Referring to Figs. 4a to 4c, in another embodiment according to the present disclosure, the transfer compartment 9 may be arranged at a middle position of the core operation module 1, the liquid operation compartment 5 and the monoclonal picking compartment 7 may be arranged at a first side of the transfer compartment 9, two purification compartments 6 may be arranged at a second side opposing the first side of the transfer compartment 9, and the loading and unloading compartment 4 may be arranged at an end of the transfer compartment 9. In this embodiment, the amplification compartment 8 may be arranged at any other suitable position. Referring to Fig. 28, in yet another embodiment according to the present disclosure, the loading and unloading compartment 4, the liquid operation compartment 5, the purification compartment 6, the monoclonal picking compartment 7, the amplification compartment 8, and the transfer compartment 9 may be in a star configuration. For example, the transfer compartment 9 may be arranged at a central position of the core operation module 1, while the loading and unloading compartment 4, the liquid operation compartment 5, the purification compartment 6, the monoclonal picking compartment 7 and the amplification compartment 8 may be arranged around the transfer compartment 9 along a circumference. In some embodiments, some spaces may also be reserved along the circumference to arrange compartments or detection instruments for other operations. For example, a separate coating compartment, PCR amplification and ELISA compartment and the like may also be arranged along the circumference.

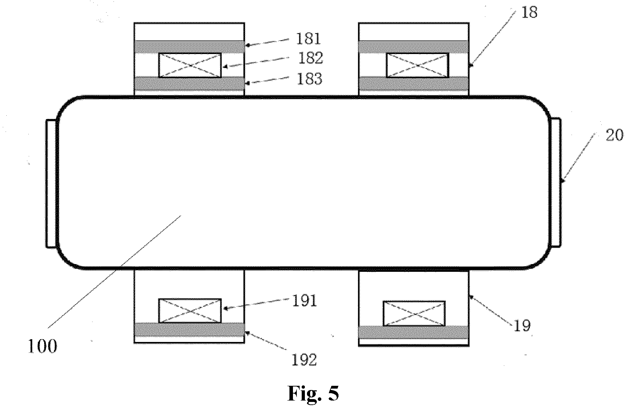

Referring to Fig. 5, in an embodiment according to the present disclosure, in order to guarantee the safety, avoid cross contamination between the samples, and avoid contamination by miscellaneous bacteria in each compartment in the operational process of gene synthesis and monoclonal picking, these compartments (the loading and unloading compartment 4, the liquid operation compartment 5, the purification compartment 6, the monoclonal picking compartment 7, the amplification compartment 8 and the transfer compartment 9) may be arranged in a compartment body 100 that can be closed tightly. The airtightness of the compartment body 100 can be effectuated by a locked sealing door 20. The compartment body 100 may include a laminar flow unit to enable each compartment to be located in a controlled laminar flow environment, so that an internal environment of the core operation module 1 is isolated from its external environment. As shown in Fig. 5, the laminar flow unit may include a laminar air-supply unit 18 and a laminar air-discharge unit 19 to form a circularly flowing laminar flow atmosphere in the compartment body 100. The laminar air-supply unit 18 may be provided on the top of the compartment body 100, while the laminar air-discharge unit may be provided at the bottom of the compartment body 100. In an embodiment according to the present disclosure, the laminar air-supply unit 18 may include an air-supply fan 182, a primary filtering unit 181, and a secondary filtering unit 182. The primary filtering unit 181 and the secondary filtering unit 182 are configured to filter out contaminants (for example, various particulate matters or other impurities) in laminar gases before the laminar gases enter the interior of the compartment body 100. The primary filtering unit 181 and the secondary filtering unit 182 may include a filter screen. The laminar air-discharge unit 19 may include an air-discharge fan 191 and a reverse filtering unit 192. The reverse filtering unit 192 is configured to prevent contaminants in the external environment from entering the interior of the compartment body 100. Likewise, the reverse filtering unit 192 may include a filter screen. In another embodiment according to the present disclosure, an ultraviolet irradiation unit may be provided in the compartment body 100 for ultraviolet disinfection of the devices in the compartment body 100. In other embodiments according to the present disclosure, the amplification compartment 8 and other incubators may not be in the laminar flow environment. Therefore, the amplification compartment 8 and other incubators may be arranged outside the compartment body 100.

Next, the specific structure of each compartment arranged in the core operation module 1 will be described in detail.

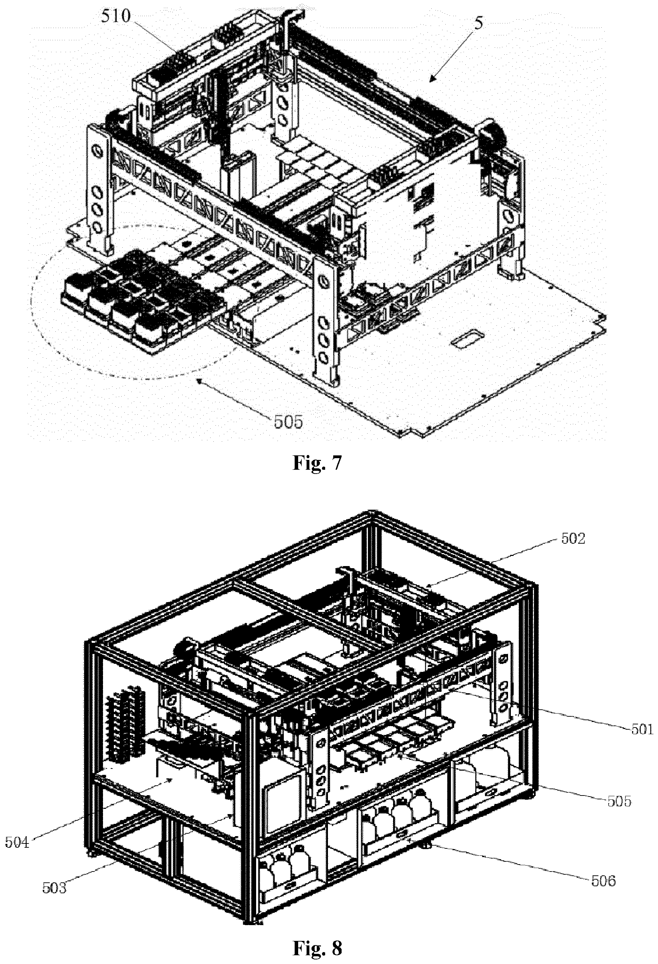

Referring first to Figs. 7 to 11, the specific structure of the liquid operation compartment 5 will be described in detail. As mentioned above, the liquid operation compartment 5 is used to perform operations such as mixing gene fragments and mixing the synthetizing reagents with the samples for synthesis, in the operational process of gene synthesis.

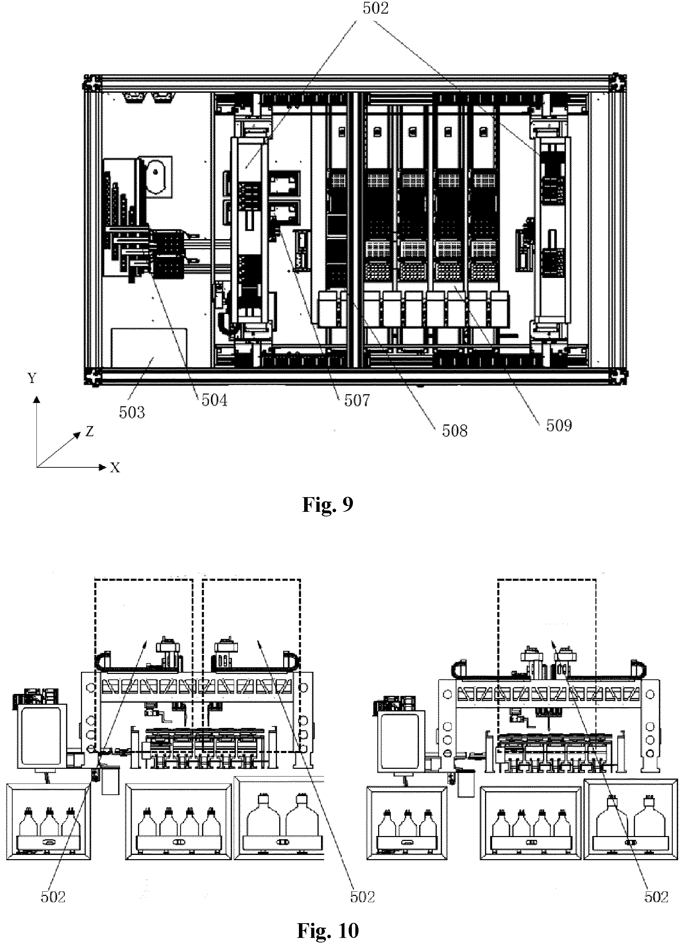

Referring to Figs. 8 and 9, in an embodiment according to the present disclosure, the liquid operation compartment 5 may include at least one pipetting unit 502, for example, one, two or more pipetting units. The pipetting unit 502 is configured to transfer the liquid required in the process of gene synthesis to perform corresponding operations. The pipetting unit 502 may be configured to be movable along an X-axis. For example, the pipetting unit 502 may be mounted on a linear motor gantry main frame structure 501 so as to be movable along the X-axis. Each pipetting unit 502 may include one or more pipetting heads 510, for example, one, two, four or more pipetting heads 510. Each pipetting head 510 can move independently along a Y-axis and a Z-axis in the pipetting unit 502, wherein the Y-axis is in a horizontal plane and perpendicular to the X-axis, and the Z-axis is in a vertical plane and perpendicular to the X-axis and the Y-axis. For example, the pipetting unit 502 may include one or more movers mounted along a direction of the Y-axis, so that each pipetting head 510 can be driven by the movers to move along the Y-axis. In addition, the pipetting unit 502 may further include a Z-axis driving mechanism to drive each pipetting head 502 to move along the Z-axis.

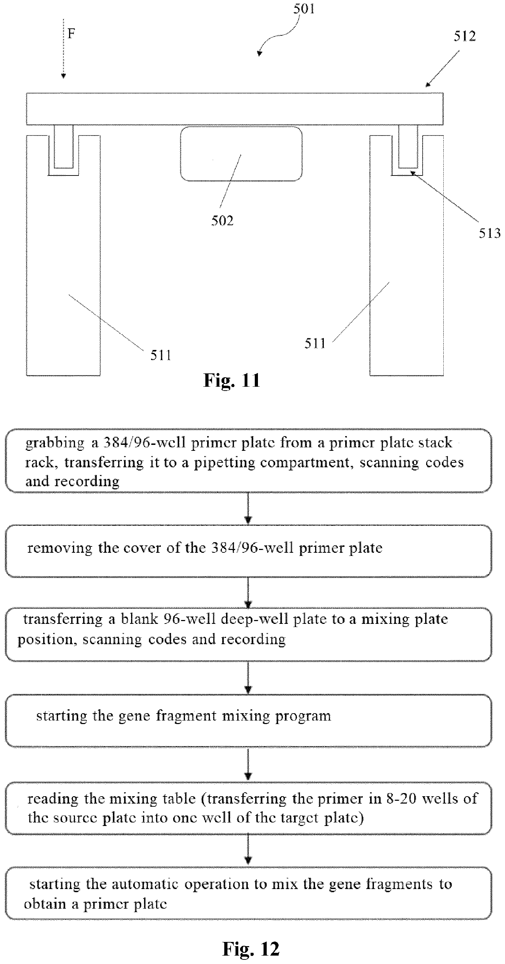

As mentioned above, the pipetting unit 502 may be mounted on the linear motor gantry main frame structure 501 so as to be movable along the X-axis. As shown in Fig. 11, the linear motor gantry main frame structure 501 may include two guide rail structures 511 spaced from each other and a cross beam 512 extending between the two guide rail structures 511. The guide rail structures 511 may extend along the X-axis, while the cross beam 512 may extend along the Y-axis. The cross beam 512 is movable along the X-axis over the guide rail structures 511. The pipetting unit 502 may be disposed on the cross beam 512 to move along the X-axis together with the cross beam 512 over the guide rail structures 511.

In an embodiment according to the present disclosure, the guide rail structures 511 and the cross beam 512 may be components of a linear motor, wherein the guide rail structures 511 may constitute a stator of the linear motor and the cross beam 512 may constitute a mover of the linear motor. With this configuration, the guide rail structures 511 and the cross beam 512 of the linear motor gantry main frame structure 501 can be connected in a non-mechanical manner. In other words, as shown in Fig. 11, a gas gap 513 (the gas gap 513 may be maintained at millimeter level) is present between the guide rail structures 511 and the cross beam 512 in the working process due to the action of electromagnetic force. This eliminates the mechanical friction between the guide rail structures 511 and the cross beam 512 as well as the dust pollution brought by mechanical friction, and this also gets rid of the requirement for grease lubrication by conventional servo mechanisms and the pollution caused by grease volatilization. On the other hand, since there is no mechanical connection between the guide rail structures 511 and the cross beam 512, the pipetting unit 502 can be easily added, reduced, replaced or repaired on the linear motor gantry main frame structure 501. For example, when there is a need to add pipetting units 502 to the linear motor gantry main frame structure 501, one or more pipetting units 502 may be conveniently mounted to the linear motor gantry main frame structure from a position above the cross beam 512 and along a direction F as shown in Fig. 11, and come into use after connection with corresponding electrical cables. When the pipetting units 502 need to be reduced, replaced or repaired, the pipetting unit 502 may be conveniently removed from a position above the cross beam 512 of the linear motor gantry main frame structure 501 and along a direction opposite to the direction F after the electrical connection is disconnected.

In an embodiment according to the present disclosure, the liquid operation compartment 5 may include a liquid pumping unit 504 and a pump set array 503. The liquid pumping unit 504 is configured to pump required process fluids into liquid storage tanks in the liquid operation compartment 5, and transfer the process fluids to the pipetting unit 502 by a robot (for example, an orifice plate robot). In an embodiment according to the present disclosure, the liquid operation compartment 5 may further comprise a bottom-compartment liquid storage unit 506 for storing process fluids and capable of performing temperature control on the process fluids. The pump set array 503 may be equipped with a multi-channel peristaltic pump or other types of pumps, for pumping the process liquids in the bottom-compartment liquid storage unit 506 into pipes of units (such as the liquid pumping unit 504 and the like) provided on the working table-board. After the liquid pumping operation, the pipes may be subjected to full-automatic cleaning and closing operation, for example, with sterilized liquid.

As mentioned above, the pipetting unit 502 is configured to transfer the liquid required in the process to consumables (such as orifice plates) to perform corresponding operations. For this purpose, the liquid operation compartment 5 may also include a corresponding operation area. In an embodiment according to the present disclosure, the liquid operation compartment 5 may include one or more of a consumable stage sliding table array 505, an oscillation unit 507, a consumable carrier 508, and an operation stage 509. The operation stage 509 is configured to perform corresponding operations on materials and can realize attitude adjustment and temporary storage of the materials during the process. The operation stage 509 may be arranged on the consumable stage sliding table array 505. The consumable stage sliding table array 505 can slide out of and into the liquid operation compartment 5 in a controlled manner, such that consumables such as orifice plates and the like can be loaded or unloaded onto or from the operation stage 509. As shown in Fig. 7, the consumable stage sliding table array 505 can slide out of the liquid operation compartment 5, so as to facilitate the loading and removal of the consumables. The oscillation unit 507 is configured to perform blending operations on the consumables such as orifice plates and the like in the process, while the consumable carrier 508 is configured to carry different types of consumables, such as orifice plates, shake tubes, shake flasks and the like.

In an embodiment according to the present disclosure, the liquid operation compartment 5 may include two or more pipetting units 502, which enables the liquid operation compartment 5 to improve the utilization efficiency of the system through real-time resource optimization. Specifically, when a small number of consumables (e.g., 6-well plate, 8-well plate, 24-well plate, etc.) are to be operated, the two or more pipetting units in the liquid operation compartment can operate independently or in parallel in two or more independent operation areas. For example, as shown in the left figure of Fig. 10, the two pipetting units 502 in the liquid operation compartment 5 can operate independently or in parallel in two independent operation areas. When the two or more pipetting units operate in parallel in their respective operation areas, the operation efficiency can be improved by orders of magnitude. When a large number of consumables (for example, 96-well plate, 384-well plate, etc.) are to be operated, the two or more pipetting units 502 may be integrated together (for example, they may be integrated into an 8-channel pipetting unit) for integral operation, as shown in the right figure of Fig. 10, thereby realizing high-throughput operation.

Fig. 12 shows an operation flow of mixing gene fragments in the liquid operation compartment 5. In this operation flow, a 96-well or 384-well primer source plate may be grabbed from a primer stack rack in the material storage module 3 and transferred to a corresponding primer source plate position in the liquid operation compartment 5, the information of the corresponding primer source plate is recorded, and then a cover of the 96-well or 384-well primer source plate may be removed. The control system 10 may be used to control the robot in the transfer compartment 9 to grab and transfer the 96-well or 384-well primer source plate and remove the cover thereof. The control system 10 may also be used to control a corresponding scanning device to scan and record the information of the corresponding primer source plate. Then, a blank 96-well or 384-well primer target plate is transferred to the mixing position, and the information of the blank 96-well or 384-well primer target plate at the mixing position is recorded (for example, using a scanning device for scanning codes and recording). Next, a gene fragments mixing program may be started, a mixing table is read, and a mixing operation is started to mix the gene fragments, thereby obtaining a primer plate with mixed gene fragments. In the mixing operation, the primers in 8-20 wells of the primer source plate may be transferred to one well of the primer target plate.

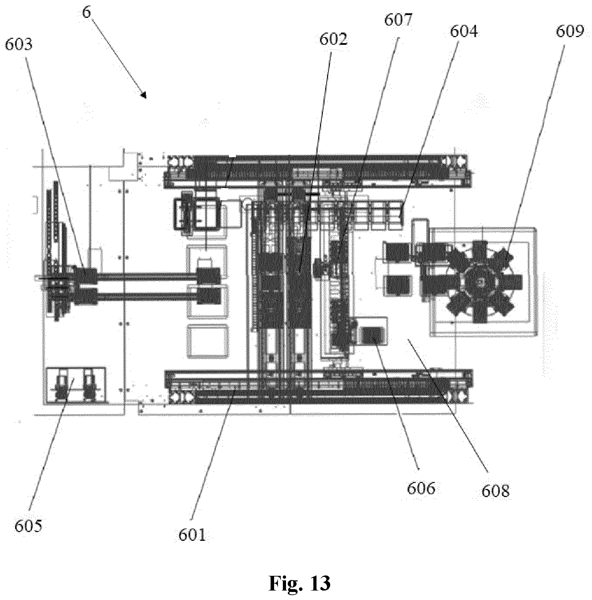

Next, with reference to Fig. 13, the specific structure of the purification compartment 6 according to an embodiment of the present disclosure will be described. Like the liquid operation compartment 5, the purification compartment 6 may include at least one purification pipetting unit 602, for example, one, two or more purification pipetting units, to transfer nucleic acid samples required for purification operation in the purification process. The purification pipetting unit 602 may include one or more purification pipetting heads, for example, one or more 96-channel purification pipetting heads, to transfer materials (for example, nucleic acid samples) between different stations. The purification pipetting head can move independently along the Y-axis and the Z-axis in the purification pipetting unit 602. The purification pipetting unit 602 may be mounted on a linear motor gantry main frame structure 601 so as to be movable along the X-axis. The linear motor gantry main frame structure 601 may have the same structure as the linear motor gantry main frame structure 501, and thus will not be described here in detail. Likewise, the purification compartment 6 may also include: a liquid pumping unit 603 for replenishing purifying reagents into reagent tanks in the purification compartment 6; a consumable stage sliding table array 604 capable of sliding out of and into the purification compartment 6 in a controlled manner so as to facilitate loading and unloading of consumables such as orifice plates; a bottom-compartment liquid storage unit 605 for storing process fluids and capable of performing temperature control on the process fluids; an oscillation unit 606 for performing mixing operation on consumables such as orifice plates during the purification operation; a consumable carrier 607 for carrying different types of consumables such as orifice plates and the like; and an operation stage 608 for attitude adjustment and temporary storage of materials during the purification operation. In an embodiment according to the present disclosure, the purification compartment 6 may further include a temperature-controlled storage device 609 for temporarily storing nucleic acid samples at a controlled temperature during operation. The temperature-controlled storage device 609 may be a 4 °C temperature-controlled storage device, so it can store nucleic acid samples at a temperature of 4 °C .

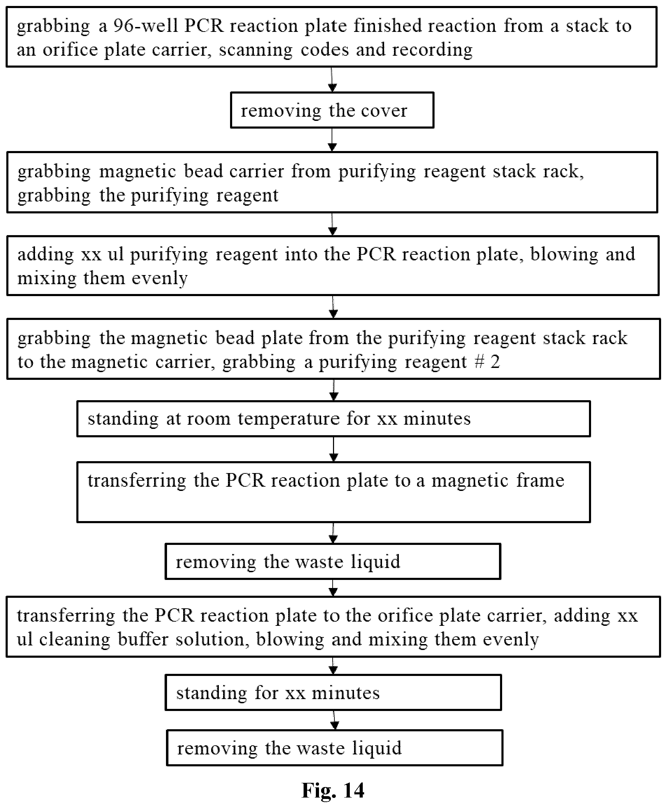

Fig. 14 shows an operation flow of performing purification operation in the purification compartment 6. This operation flow may comprise: grabbing a PCR reaction orifice plate, which has finished reaction, from a PCR reaction orifice plate stack rack first and transferring it to an orifice plate carrier, scanning codes and recording the PCR reaction orifice plate, and opening a cover of the PCR reaction orifice plate; then, grabbing a magnetic bead plate from a purifying reagent stack rack and transferring it to a magnetic bead carrier; acquiring a purifying reagent; adding a predetermined amount of the purifying reagent into the PCR reaction orifice plate and mixing them uniformly. In some cases, the above operation steps may be repeated until all of the PCR reaction orifice plates are filled with a predetermined amount of the purifying reagent; next, keeping the uniformly-mixed PCR reaction orifice plates at room temperature for a predetermined period of time and then transferring them to a magnetic frame to remove waste liquids; later, transferring the PCR reaction orifice plates having the waste liquids removed to the PCR reaction orifice plate stack rack, adding a predetermined amount of cleaning buffer solution into them and mixing them uniformly, and then standing them for a predetermined time to remove further waste liquids from the PCR reaction orifice plates, thus completing the purification operation.

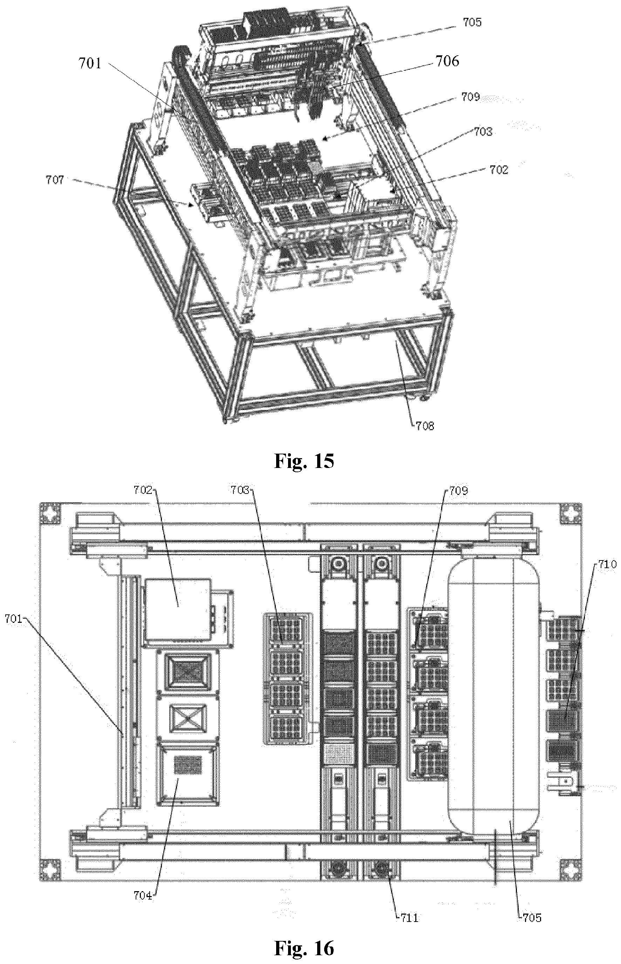

With reference to Figs. 15 to 16, the specific structure of the monoclonal picking compartment 7 according to an embodiment of the present disclosure will be described. The monoclonal picking compartment 7 may include a monoclonal picking unit 702 for picking biological samples such as monoclonal bacterial strains or cell strains from the orifice plate (for example, an agar plate). The monoclonal picking unit 702 may be mounted on a linear motor gantry main frame structure 701 to be transferred to a target orifice plate position. The linear motor gantry main frame structure 701 may be an X-Y linear motor gantry main frame structure, so as to be able to transfer the monoclonal picking unit 702 along the X-axis and the Y-axis. The linear motor gantry main frame structure 701 may have the same structure as the linear motor gantry main frame structure 501 and thus will not be described here in detail.

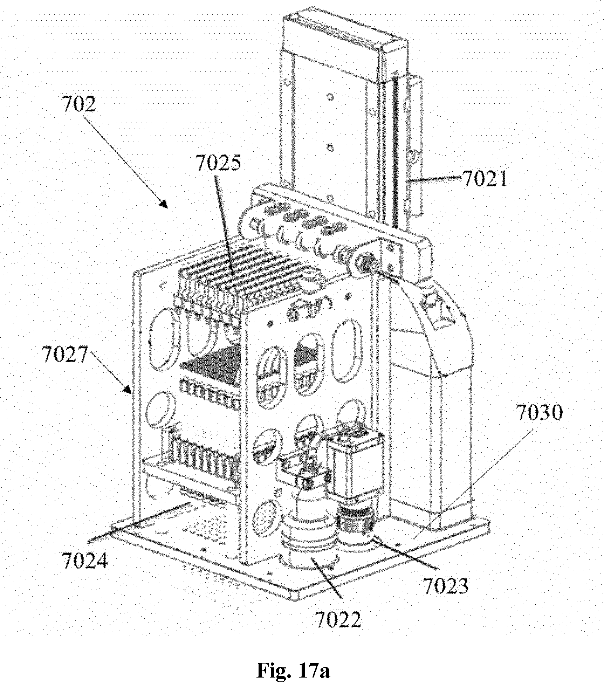

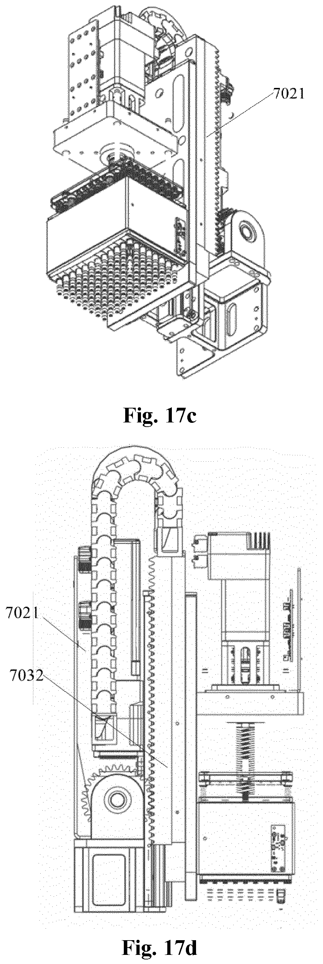

Referring to Figs. 17a to 17d, the specific structure of the monoclonal picking unit 702 according to an embodiment of the present disclosure is shown. The monoclonal picking unit 702 may include a multi-channel pick head assembly 7026 (for example, 4-, 8-, 16-, 24-, 48- or 96-channel pick head assembly) formed by integrating a plurality of (for example, 4, 8, 16, 24, 48 or 96) pick head elements 7024 together. The multi-channel pick head assembly may be connected to an integrated gas path on-off valve group 7025 so as to carry out picking operation under the control of the integrated gas path on-off valve group 7025. The integrated gas path on-off valve group 7025 can control each pick head element 7024 separately, so that each pick head element 7024 is capable of independently moving and fulfilling the operation of picking monoclonal bacterial strains or cell strains independently. Of course, in other embodiments according to the present disclosure, the monoclonal picking unit 702 may also include only one picking unit 7024, thus forming a single-channel pick head assembly.

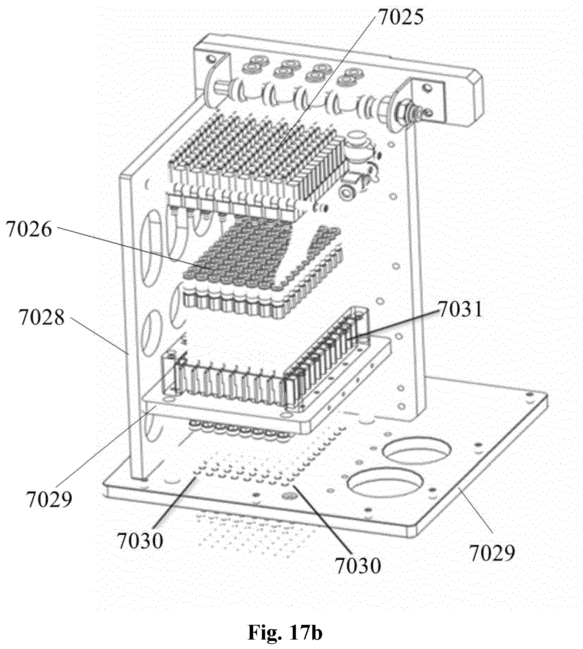

In an embodiment according to the present disclosure, the monoclonal picking unit 702 may include a Z-axis motor 7021. The multi-channel pick head assembly can be driven by the Z-axis motor 7021 to move up and down along a direction of the Z-axis. As shown more clearly in Figs. 17b and 17c, the Z-axis motor 7021 can drive the multi-channel pick head assembly to move up and down along the direction of the Z-axis via a gear-rack mechanism 7032.

In an embodiment according to the present disclosure, in order to align the multi-channel pick head assembly with a target orifice plate to be picked to carry out the picking operation, the monoclonal picking unit 702 may include an image capturing assembly 703 for identifying and localizing the target biological sample such as the target bacterial strain or the target cell strain in the monoclonal picking process. The image capturing assembly 703 may include a camera 7023. The camera can capture images of the target orifice plate in real time to determine the position of the biological sample such as the target bacterial strain or the target cell strain to be picked. The camera, after having determined the position of the biological sample such as the target bacterial strain or the target cell strain to be picked, can also send a position adjustment signal to a linear motor provided on the linear motor gantry main frame structure 701, so as to move the multi-channel pick head assembly to the target position. The image capturing assembly 703 may also include an optical distance sensor 7022. The optical distance sensor can be used to calibrate a vertical distance between tip ends of the pick head elements in the multi-channel pick head assembly and a surface of the target orifice plate, and to evaluate a surface flatness of the target orifice plate. The optical distance sensor, after having calibrated the vertical distance between the tip ends of the pick head elements in the multi-channel pick head assembly and the surface of the target orifice plate, can send a height adjustment signal to the Z-axis motor 7021 to move the multi-channel pick head assembly up and down along the direction of the Z-axis to a predetermined height.

As shown more clearly in Figs. 17a and 17b, the monoclonal picking unit 702 may include a bracket assembly 7027. The bracket assembly 7027 may include vertical support plates 7028 and a plurality of horizontal support plates 7029 extending perpendicular to the vertical support plate 7028. Each horizontal support plate 7029 is provided with holes through which the plurality of pick head elements 7024 extend, to ensure the accurate localization of the plurality of pick head elements 7024. In an embodiment according to the present disclosure, one of the plurality of horizontal support plates 7029 is further provided with a guiding and positioning mechanism 7031 for the plurality of pick head elements 7024, in order to localize and guide the vertical movement of the plurality of pick head elements 7024 in the multi-channel pick head assembly 7026. In another embodiment according to the present disclosure, an upper surface 7030 of a bottom horizontal support plate of the plurality of horizontal support plates 7029 functions as a limiting surface for each pick head element 7024 in the multi-channel pick head assembly 7026, in order to ensure that the tip ends of the plurality of pick head elements 7024 are at a same height. The upper surface of the bottom horizontal support plate may be formed in a single processing step to ensure its accuracy, so that the tip end of each pick head element 7024 can be positioned more accurately, and the tip ends of the plurality of pick head elements 7024 can be arranged at the same height more accurately. Further, as shown in Fig. 17b, the optical distance sensor 7022 may also be arranged on the upper surface 7030 of the bottom horizontal support plate so as to accurately calibrate the vertical distance between the tip ends of the pick head elements 7024 in the multi-channel pick head assembly 7026 and the surface of the target orifice plate. Similarly, the camera 7023 may also be arranged on the upper surface 7030 of the bottom horizontal support plate.

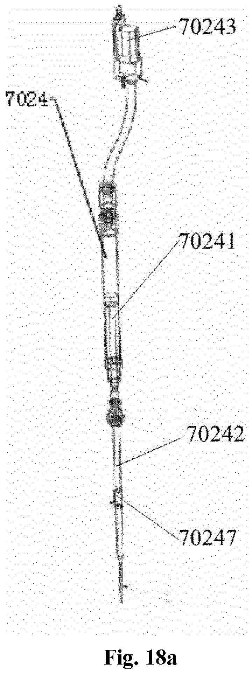

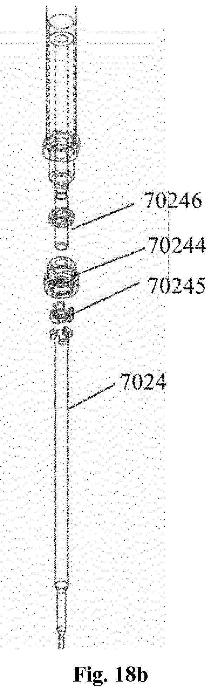

Referring to Figs. 18a and 18b, the specific structure of the pick head element 7024 according to an embodiment of the present disclosure will be described in detail. As shown in Figs. 18a and 18b, the pick head element 7024 may include a first section 70241 and a second section 70242. An upper end of the first section 70241 is connected with a drive control valve 70243 in the integrated gas path on-off valve group 7025, such that the vertical movement of the pick head element 7024 can be driven by the drive control valve 70243. A lower end of the first section 70241 and an upper end of the second section 70242 are configured in a floating connection to ensure that the tip ends of the plurality of pick head elements 7024 can be more easily positioned at the same height, which allows a reduction in the assembling difficulty of the multi-channel pick head assembly 7026 and a reduction in the machining accuracy of the bracket assembly 7027. As shown more clearly in Fig. 18b, the pick head element 7024 may include a floating connection mechanism to realize the floating connection between the lower end of the first section 70241 and the upper end of the second section 70242. The floating connection mechanism may include a sleeve element 70244 and a floating element 70245. The floating element 70245 may be configured as a floating ferrule and may be fixedly connected with the upper end of the second section 70242. The floating element 70245 is configured to be mountable in the sleeve element 70244 and capable of floating a distance within the sleeve element 70244 (for example, floating up and down over a distance in the vertical direction). The lower end of the first section 70241 may also be fixed in the sleeve element 70244, thus achieving a floating connection with the second section 70242. For this purpose, the lower end of the first section 70241 may include a threaded joint 70246 for threadedly connecting the lower end of the first section 70241 to the sleeve element 70244.

In an embodiment according to the present disclosure, the pick head element 7024 may further include a guide element 70247. The guide element 70247 may be disposed on an outer periphery of the second section 70242 of the pick head element 7024. The guide element 70247 may be configured as a guide bearing. When the pick head element 7024 is mounted on the bracket assembly 7027, the guide element 70247 may extend through the guiding and positioning mechanism 7031 provided on one of the horizontal support plates to guide the vertical movement of the pick head element 7024. The guide element 70247 may be made of a wear-resistant material to prevent the occurrence of dust and particle contamination caused by friction during movement. In an embodiment according to the present disclosure, the tip end of the second section 70242 of the pick head element 7024 is used to perform the picking operation. The tip end of the pick head element 7024 may be appropriately shaped to reach the best picking effect and transfer safety, and to be prevented from falling during transfer.

In the embodiment according to the present disclosure, the pick head element 7024 may be made of SUS316 stainless steel material that meets GMP requirements to maintain the cleanliness of the picked sample. In the embodiment according to the present disclosure, the adoption of the bracket assembly 7027 can realize modular design of the monoclonal picking unit 702. This not only allows any arbitrary integration from a single-channel pick head assembly to any number of the multi-channel pick head assemblies, but also can ensure the accuracy of the relative position between the pick head elements in the multi-channel pick head assembly.

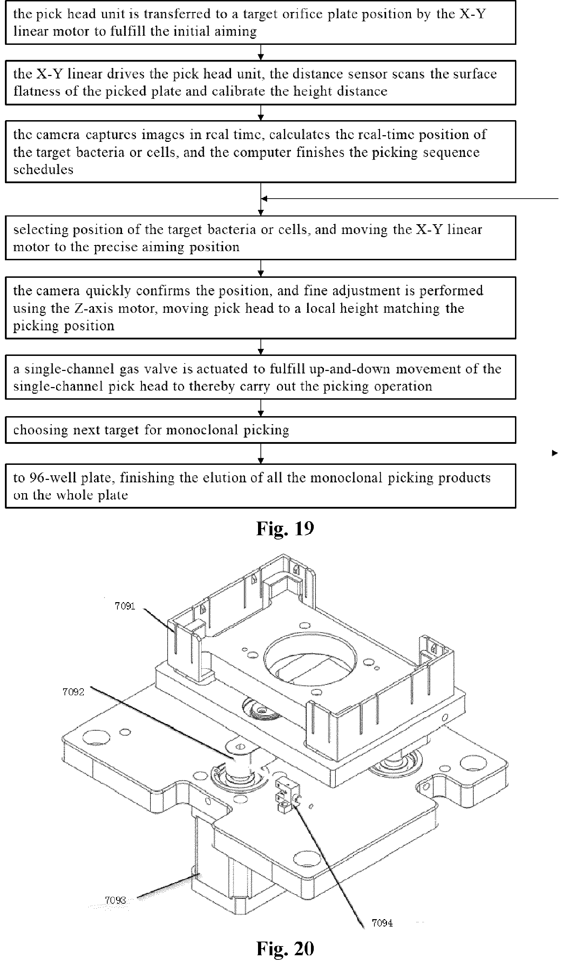

Fig. 19 shows an operation flow of monoclonal picking with the monoclonal picking unit 702. In this operation flow, firstly, the monoclonal picking unit 702 is transferred to a target orifice plate position by a linear motor provided on the linear motor gantry main frame structure 701 to complete the preliminary aiming operation. Then, the height distance (that is, the vertical distance between the tip ends of the pick head elements in the multi-channel pick head assembly and the surface of the target orifice plate to be picked) of the target orifice plate to be picked is calibrated and its flatness is scanned by using the optical distance sensor 7022 of the monoclonal picking unit 702. Meanwhile or later, the camera 7023 may be used to capture the image of the target orifice plate to be picked in real time to real-time calculate the position of the target bacterial strain or cell strain and transmit the calculated result to the control system, which sets the picking sequence schedule based on the real-time position of the target bacterial strain or cell strain. Thereafter, the control system selects the position of the target bacterial strain or cell strain to be picked, and sends a position adjustment signal to the linear motor provided on the linear motor gantry main frame structure 701, so as to move the multi-channel pick head assembly of the monoclonal picking unit 702 to a position precisely aligned with the target bacterial strain or cell strain to be picked. At this time, the position of the target bacterial strain or cell strain to be picked can be re-determined using the camera 7023, and fine adjustment may be performed using the Z-axis motor 7021 to move the multi-channel pick head assembly to a predetermined height matching the picking position. Next, a single one of the drive control valve 70243 in the integrated gas path on-off valve group 7025 may be started to allow the corresponding single pick head element 7024 to move up and down under the control of the single drive control valve 70243 to thereby carry out the picking operation. After one target has undergone monoclonal picking, another target is selected for monoclonal picking, and one or more of the above steps may be repeated until a predetermined number of targets have been subjected to monoclonal picking. After a predetermined number of targets have been subjected to monoclonal picking, the multi-channel pick head assembly may be moved to a multi-well orifice plate (for example, a 96-well plate), and then all the biological samples picked are eluted to said multi-well orifice plate.

Returning to Figs. 15 to 16, in an embodiment according to the present disclosure, in order to facilitate the monoclonal picking of the gene-synthesized biological samples, the monoclonal picking compartment 7 may further include an oscillation-coating assembly 709 for realizing uniform distribution of flora on the orifice plate (e.g., an agar plate) so as to enable high-efficiency culturing of the monoclonal flora and prepare for high-throughput monoclonal picking. Further, the monoclonal picking compartment 7 may further include a multi-channel pipetting unit 705 and a liquid pumping unit 706. The multi-channel pipetting unit 705 is configured to transfer fluids between different operating positions, for example, it may be used to pick liquids such as reagents and culture media and fill them into the wells of the orifice plate, and it may also be used to transfer synthesized biological samples between different operating positions. The multi-channel pipetting unit 705 may include different numbers of channels based on the required process. As shown more clearly in Fig. 15, the multi-channel pipetting unit 705 may also be arranged on the linear motor gantry main frame structure 701 to be moved along the X-axis and/or the Y-axis under the drive of the linear motor in the linear motor gantry main frame structure 701 to thereby transfer fluids between different stations. The liquid pumping unit 706 is used to add fluids such as culture media and picking reagents into the reagent tank of the monoclonal picking compartment 7.

Fig. 20 shows the specific structure of the oscillation-coating assembly 709 according to an embodiment of the present disclosure. As shown in Fig. 20, the oscillation-coating assembly 709 may include a clamping mechanism 7091 and a cam oscillating mechanism 7092. The orifice plate (for example, an agar plate) may be clamped in the clamping mechanism 7091 and oscillated together with the clamping mechanism 7091 under the drive of the cam oscillating mechanism 7092, so as to realize the uniform distribution of flora on the orifice plate. The cam oscillating mechanism 7092 may be driven by a drive motor 7093. Further, the oscillation-coating assembly 709 may further include an in-situ sensor 7094 for detecting a reference position of the oscillation-coating assembly 709.

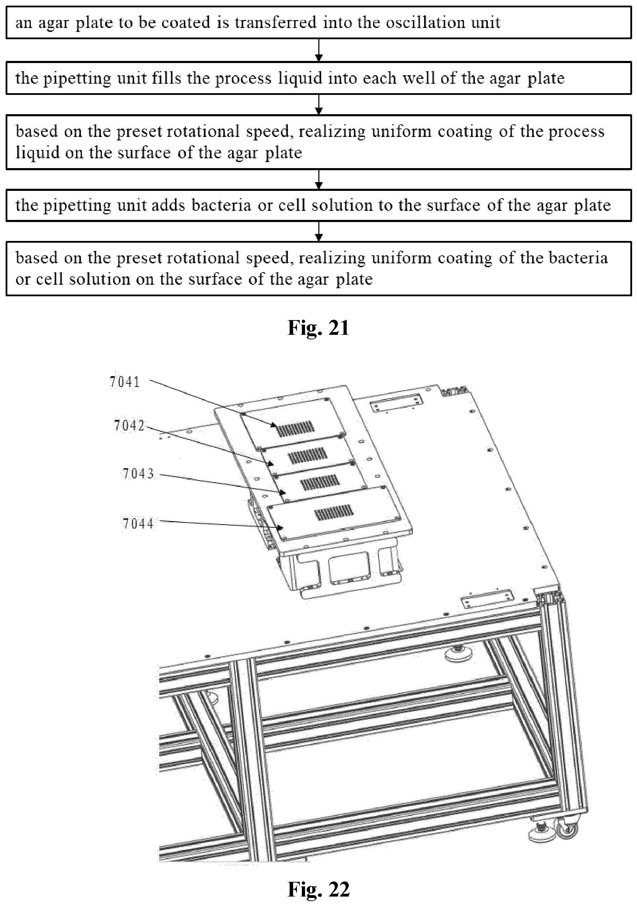

Fig. 21 shows an operation flow of coating flora on an orifice plate with the oscillation-coating assembly 709 according to an embodiment of the present disclosure. In this operation flow, the orifice plate (for example, an agar plate) to be coated is transferred to the oscillation-coating assembly 709 first and clamped in the clamping mechanism 7091. Then, a process fluid is filled in each well of the orifice plate using the multi-channel pipetting unit 705, and the rotational speed of the cam oscillating mechanism 7092 is set to drive the clamping mechanism 7091 and the orifice plate therein to oscillate at the set rotational speed, thereby realizing uniform coating of the process fluid on the surface of the orifice plate. Then, a cell/bacteria solution is added onto the surface of the orifice plate using the multi-channel pipetting unit 705, and the rotational speed of the cam oscillating mechanism 7092 is set to drive the clamping mechanism 7091 and the orifice plate therein to oscillate at the set rotational speed, thereby realizing uniform coating of the cell/bacteria on the surface of the orifice plate.

Referring to Figs. 16 and 22, after each batch of the monoclonal picking, the multi-channel pick head assembly of the monoclonal picking unit 702 needs to be deeply cleaned (e.g., sterilized) to avoid cross-contamination between various batches of the monoclonal picking. For this purpose, in an embodiment according to the present disclosure, a pick head cleaning assembly 704 may be provided in the monoclonal picking compartment 7. As shown clearly in Fig. 22, the pick head cleaning assembly 704 may include a plurality of pick head cleaning elements, such as a first pick head cleaning element 7041, a second pick head cleaning element 7042, and a third pick head cleaning element 7043 as shown in Fig. 22. Each of the pick head cleaning elements may contain liquids (such as alcohol, etc.) for cleaning the pick head. With the help of the plurality of pick head cleaning elements, the multi-channel pick head assembly of the monoclonal picking unit 702 can be deeply cleaned.

After the deep cleaning of the multi-channel pick head assembly of the monoclonal picking unit 702, it is necessary to effectively remove the residual cleaning liquid (such as alcohol, etc.) on the pick head and ensure that the cleaned pick head is at a normal temperature to prevent the residual cleaning liquid from killing the target cells/bacteria, thus ensuring the safety of the target cells/bacteria. For this purpose, the pick head cleaning assembly 704 may further include a pick head drying element 7044. The pick head drying element 7044 may include a heating unit to ensure quick drying of the pick head element. In addition, the pick head drying element 7044 may also include an air-supply duct and an air-discharge duct to realize rapid cooling of the pick head elements that have been dried.

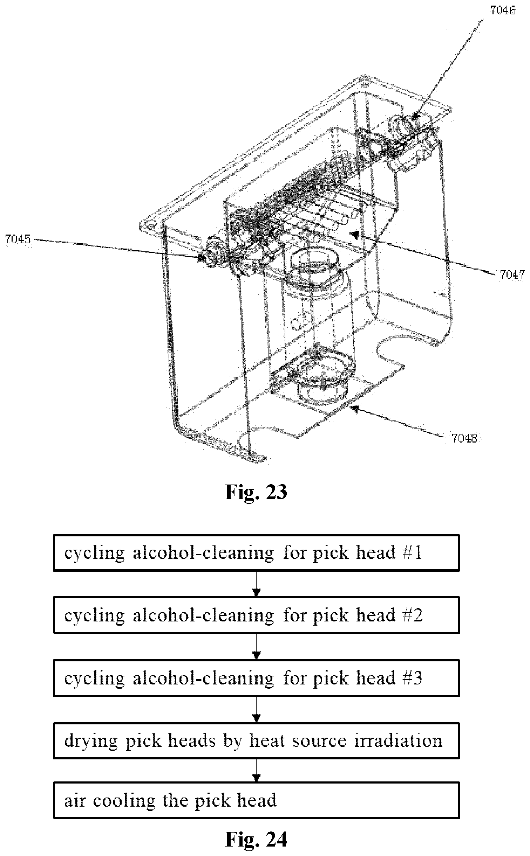

Fig. 23 shows the specific structure of the pick head drying element 7044 according to an embodiment of the present disclosure. In this embodiment, the pick head drying element 7044 may include a heating unit 7047. The heating unit 7047 may include a plurality of heating elements, such as electric heating wires, electric heating rods, irradiation heating lamps, and the like. Further, as shown in Fig. 23, the pick head drying element 7044 may include two air inlets 7045 and 7046 provided on the left and right sides thereof, and an air outlet 7048 arranged in a vertical direction between the two air inlet holes 7045 and 7046. The air inlets 7045 and 7046 and the air outlet 7048 are configured to be in fluid communication with each other, so that air can enter the interior of the pick head drying element 7044 through the air inlets 7045 and 7046 and be discharged from the interior of the pick head drying element 7044 through the air outlet 7048, thereby realizing rapid air cooling of the pick head. The pick head drying element 7044 may include an air-supply device to send air into the interior of the pick head drying element 7044 through the air inlets 7045 and 7046.

Fig. 24 shows an operation flow of cleaning the pick head element with the pick head cleaning assembly 704 according to an embodiment of the present disclosure. In this operation flow, first, a plurality of pick head cleaning elements 7041, 7042 and 7043 may be used to clean the pick head elements in turn, and then the cleaned pick heads are transferred to the pick head drying element 7044. In the pick head drying element 7044, the cleaned pick head elements are first heated by the heating unit 7047 to evaporate the cleaning liquid remaining on the pick head element, and then air is sent into the interior of the pick head drying element 7044 by the air-supply device to cool the heated pick head elements to return them to the normal temperature.

Returning to Figs. 15 and 16, similar to the liquid operation compartment 5 and the purification compartment 6, the monoclonal picking compartment 7 may also include: a bottom-compartment liquid storage unit 708 for storing process fluids and capable of performing temperature control on the process fluids; a consumable stage sliding table array 707 capable of fully automatically sliding in and out of the monoclonal picking compartment 7, so as to facilitate the loading of consumables such as orifice plates, reagent tanks and the like; a consumable carrier 710 for carrying different types of consumables such as orifice plates, shake tubes, shake flasks and the like according to actual process requirements; an operation stage 711 used to perform corresponding operations on materials and capable of realizing attitude adjustment and temporary storage of the materials during the process. Further, the monoclonal picking compartment 7 may further include a temperature-controlled storage device 712 for temporarily storing nucleic acid samples at a controlled temperature during operation. The temperature-controlled storage device 712 may be a 4 °C temperature-controlled storage device, so it can store nucleic acid samples at a temperature of 4 °C.

As described above, the intelligent system for gene synthesis and monoclonal picking according to the present disclosure may include the control system 10. Fig. 25 shows a basic architecture of the control system 10. The control system 10 may include a control computer 101, an I/O data collecting module 102, and a drive module 103. The I/O data collecting module 102 can collect various required data, including but not limited to temperature, humidity, pressure, weight, distance, etc., by means of various sensors, and send the collected data to the control computer 101. The control computer 101 can execute calculation based on various data collected by the I/O data collecting module 102, and issue various instructions to control the drive module 103 to perform various operations. For example, the control computer 101 may control the drive module 103 to execute control on pumps, valves, temperature as well as other actuating mechanisms. Said other actuating mechanisms include, but are not limited to, rotary motors, linear motors, electric sliding tables, cameras, and the like. The control system 10 may also include a laboratory data management system (LDMS)104, which may interact with the control computer 101 to store and/or read various necessary data.

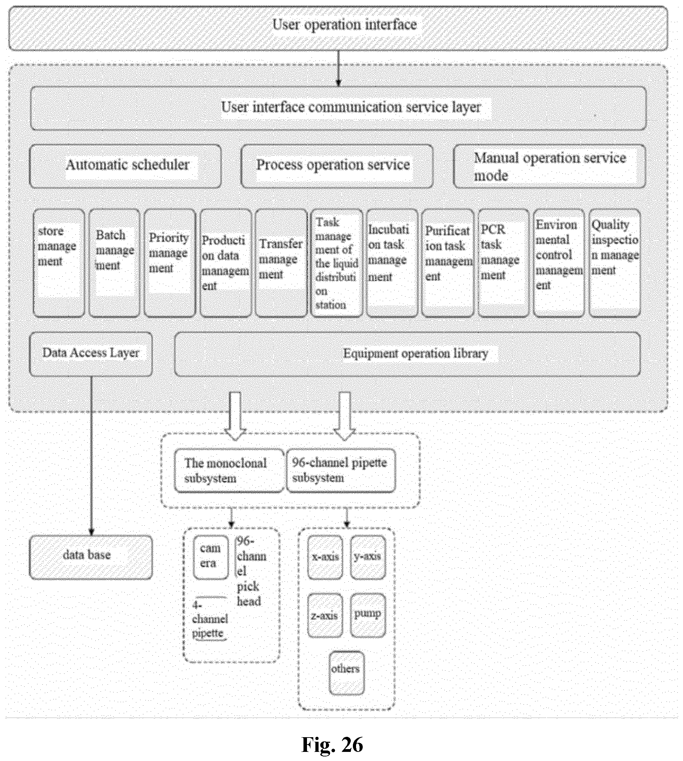

As shown in Fig. 25, in an embodiment according to the present disclosure, the control computer of the control system 10 may be equipped with a comprehensive software system 11 to ensure the full-automatic operation of the whole intelligent system for gene synthesis and monoclonal picking and realize the collection, storage and analysis of data during the operation. As shown in Fig. 27, the software system 11 may include, but is not limited to, the following units:
1) a workflow editing unit 12, which provides workflow editing functions, including but not limited to: editing and managing a full-flow process list, providing a standardized process template, and controlling and adjusting the process in real time;
2) a device monitoring and operating unit 13, which may be in a full-automatic operation mode and at least provides the following functions: managing the historical data of sample liquids operation, the historical data of sample magnetic beads operation, the historical data of sample coating operation, the historical data of sample monoclonal picking and the historical data of sample transferring operation, managing the state of materials at each station, monitoring the running state of modules of each device, managing the loading of solid consumables, managing the loading of liquid materials, managing the storage of consumables at the storage center, and managing the replenishment of consumables at the storage center;
3) a device debugging unit 14, which may be in a manual operation mode and provides at least the following functions: calibrating and correcting each mobile module in the system, calibrating and correcting each liquid unit in the system, demonstrating the intelligent monoclonal sample picking, and demonstrating the sample coating process;
4) a data storage unit 15, which can provide at least the following functions: storing the original image data of monoclonal picking;
5) a log prompting unit 16, which can provide at least the following functions: recording the operator's login information, material loading information, device failure information during operation, device warning information during operation, and device operation information during operation; and
6) a parameter setting unit 17, which can provide at least the following functions: setting personnel's authority, setting parameters of each module in the system, configuring the types of liquid pipes, configuring the capacity of liquid, configuring the operation parameters of liquid units, configuring the intelligent image recognition of monoclonal samples, and configuring the coating movement parameters. Fig. 26 shows a basic architecture of the software system 11 according to an embodiment of the present disclosure.

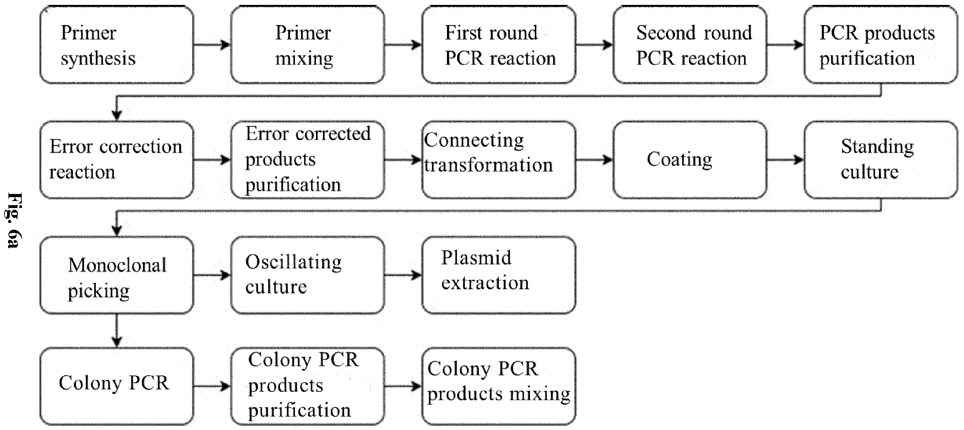

Return to Figs. 6a-6b, which show various typical process flows of gene synthesis and monoclonal picking that can be performed using the system for gene synthesis and monoclonal picking or its core operation module 1 according to the present disclosure.

As shown in Fig. 6a, in an embodiment according to the present disclosure, first, primer synthesis and primer mixing may be carried out in the liquid operation compartment 5, then one or more rounds of PCR reaction (for example, two rounds of PCR reaction shown in Fig. 6a) may be carried out in the amplification compartment 8 to obtain a PCR product, which is subsequently subjected to purifying operation in the purification compartment 6. Later, the purified PCR product may be subjected to an error correction reaction to obtain an error corrected product, and the error corrected product may be purified in further. Next, the error corrected product after purification may be subjected to connecting transformation (for example, connecting the primer fragments to vectors), coating, standing culture, and monoclonal picking operations to pick out the required cells/bacteria. The cells/bacteria picked out may be operated in two routes. In one of the two routes: the cells/bacteria picked out continue to be subjected to colony PCR operation to obtain colony PCR products; purification operation is performed on the colony PCR products to realize mixing of the purified colony PCR products; and then the purified colony PCR products can be subjected to high-throughput sequencing (NGS sequencing). In the other one of the two routes: the cells/bacteria picked out are directly subjected to oscillating culture, and then plasmid extraction is performed on the oscillating-cultured cells/bacteria.

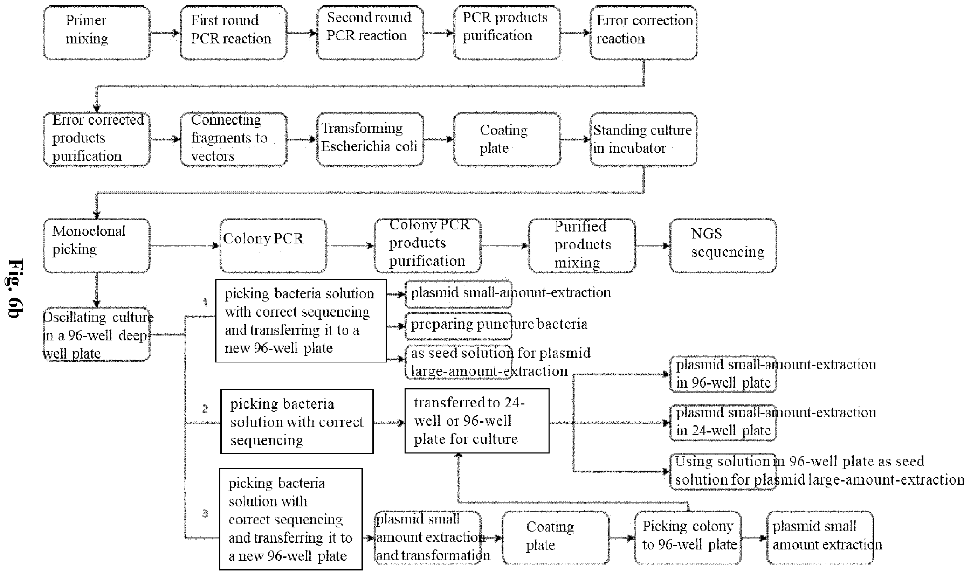

As shown in Fig. 6b, when performing oscillating culture, the cells/bacteria obtained by monoclonal picking may be transferred to a porous deep-well plate for the oscillating culture. After oscillating culture, one or more of the following operations may be carried out as required: 1) picking a bacteria solution with correct sequencing and transferring it to a new multi-well plate (for example, a 96-well plate), and then performing plasmid small-amount-extraction, preparing puncture bacteria, or using it as a seed solution for plasmid large-amount-extraction; 2) picking a bacteria solution with correct sequencing and transferring it to a multi-well plate (such as a 24-well plate or a 96-well plate) for culture, and then performing plasmid small-amount-extraction in the multi-well plate (such as the 24-well plate or the 96-well plate) or using the bacteria solution as a seed solution for plasmid large-amount-extraction; and 3) picking a bacteria solution with correct sequencing and transferring it to a new multi-well plate (for example, a 96-well plate), performing plasmid small-amount-extraction and transformation, and then, subjecting the transformed product to coating and monoclonal picking, and transferring it to a multi-well plate for plasmid small-amount-extraction and the like.

According to the present disclosure, by adopting the intelligent operation of gene synthesis process from primer synthesis to monoclonal picking, the amount of personnel required in the process from gene synthesis to monoclonal picking can be reduced by orders of magnitude and the processing throughput in the whole process can be increased by orders of magnitude, thereby meeting the requirements for efficiency and effectiveness in the process of research, development and evaluation of new drugs and effectively reducing costs. Meanwhile, it can also realize the intensive use of core device units in the process and reduce the requirements for use space by orders of magnitude. In addition, the standardized system and supporting technology facilitate the flexible arrangement of the system and the promotion of the standardized method in different locations. Finally, replacement of the manual operation with the automatic system can solve problems related to stability, uniformity and reproducibility in the operation process.

According to the present disclosure, by adopting the laminar flow unit, the biological safety of the samples, environment and operators can be effectively guaranteed, and confusion and cross-contamination between the samples can be strictly avoided in the operation process. Further, the adoption of the software system enables standardized collection, storage and analysis of data in the operation process, not only improving the efficiency of analysis and screening, but also realizing intelligent docking with upstream and downstream automatic systems.

Other embodiments of the present disclosure will be disclosed below.

Referring to Figs. 1-27, the present disclosure also provides a technical solution as following:

An intelligent system for gene synthesis and monoclonal picking comprises a core operation module 1, a sequencer module 2, a material storage module 3 and a control computer 10. The core operation module 1 includes a loading and unloading compartment 4, a liquid operation compartment 5, a purification compartment 6, a monoclonal picking compartment 7, an amplification compartment 8 and a transfer compartment 9. The liquid operation compartment 5 includes a linear motor gantry main frame 501, a pipetting unit 502, a pump set array 503, a unit for pumping and replenishing liquid 504, a first consumable stage sliding table array 505, a first bottom-compartment liquid storage unit 506, a first oscillation unit 507, a first consumable carrier 508 and a first operation stage 509. The purification compartment 6 includes a first X-Y linear motor gantry structure 601, a 96-channel purification pipetting head 602, a first liquid pumping unit 603, a second consumable stage sliding table array 604, a second bottom-compartment liquid storage unit 605, a second oscillation unit 606, a second consumable carrier 607, a second operation stage 608 and a first 4 °C temperature-controlled storage device 609. The monoclonal picking compartment 7 includes a second X-Y linear motor gantry structure 701, a 96-channel monoclonal picking unit 702, an orifice plate image capturing assembly 703, a pick head sterilizing and cleaning assembly 704, a multi-channel pipetting unit 705, a second liquid pumping unit 706, a third consumable stage sliding table array 707, a third bottom-compartment liquid storage unit 708, an oscillation-coating assembly 709, a third consumable carrier 710, a third operation stage 711 and a second 4 °C temperature-controlled storage device 712. A comprehensive control software 11 is installed in the control computer 10. The control software 11 is provided therein with a workflow editing unit 12, a full-automatic device monitoring and operating unit 13, a manual device debugging unit 14, a data storage unit 15, a log prompting unit 16 and a parameter setting unit 17. The loading and unloading compartment 4, the liquid operation compartment 5, the purification compartment 6, the monoclonal picking compartment 7, the amplification compartment 8 and the transfer compartment 9 are each provided with laminar air-supply units 18 on both sides of their top's center. The loading and unloading compartment 4, the liquid operation compartment 5, the purification compartment 6, the monoclonal picking compartment 7, the amplification compartment 8 and the transfer compartment 9 are each provided with laminar air-discharge units 19 on both sides of their bottom's center. The loading and unloading compartment 4, the liquid operation compartment 5, the purification compartment 6, the monoclonal picking compartment 7, the amplification compartment 8 and the transfer compartment 9 are each provided with locked sealing door bodies 20 on their both sides. The laminar air-supply unit 18 includes a primary filtering unit 181, an air-supply fan 182, and a secondary filtering unit 183. The laminar air-discharge unit 19 includes an exhaust fan 191 and a reverse filtering unit 192.

Referring to Figs.7-12, when consumables such as a 24-well plate, a 6-well plate and an 8-well plate are operated in the liquid operation compartment 5, the two pipetting units 502 operate in parallel in the left and right independent areas, which is equivalent to two pipetting units 502 working in parallel, capable of improving operation efficiency by orders of magnitude. When operation is performed on a 96/384-well plate, the two pipetting units 502 are integrated into an 8-channel pipetting unit, which can operate on the 96/384-well plate. There is no mechanical connection between the X-axis guide rail and the Y-axis cross beam in the linear motor gantry main frame structure 501. When functions are to be added, a new module can be conveniently mounted from a position above the gantry structure and can come into use only by connection with electrical cables; when the working unit needs to be replaced or repaired, the working unit can be conveniently removed from a position above the gantry after the electrical connection is disconnected. During operation, a millimeter-level gas gap is maintained between the X-axis guide rail and the Y-axis cross beam, which eliminates the mechanical friction and the resultant dust pollution, and at the same time gets rid of the requirement for grease lubrication by conventional servo structures and the pollution caused by grease volatilization. A typical automatic operation flow of mixing primer in the liquid operation compartment 5 is as follows: firstly, grabbing a 384/96-well primer plate from a primer plate stack rack, transferring it to a pipetting compartment, scanning codes and recording; removing a cover of the 384/96-well primer plate, transferring a blank 96-well deep-well plate to a mixing plate position, scanning codes and recording; finally, starting the gene fragment mixing program, reading the mixing table, transferring the primer in 8-20 wells of the source plate into one well of the target plate, and starting the automatic operation to mix the gene fragments to thereby obtain a primer plate.

Referring to Figs. 13 and 14, a typical automatic operation flow of purifying nucleic acids in the purification compartment 6 is as follows: firstly, grabbing a 96-well PCR reaction plate, which has finished reaction, from a stack rack and transferring it to an orifice plate carrier, scanning codes and recording, and removing the cover thereof; then grabbing a magnetic bead carrier from a purifying reagent stack rack and meanwhile grabbing the purifying reagent; adding an amount of (ul) purifying reagent into the PCR reaction plate, and blowing and mixing them evenly; later, grabbing the magnetic bead carrier from the purifying reagent stack rack and meanwhile grabbing a purifying reagent # 2; standing at room temperature for certain minutes; transferring the PCR reaction plate to a magnetic frame to remove the waste liquid; finally, transferring the PCR reaction plate to the orifice plate carrier, adding an amount of (ul) cleaning buffer solution, and blowing and mixing them evenly; and removing the waste liquid after standing for certain minutes.

Referring to Figs. 15-24, the 96-channel monoclonal picking unit 702 includes a Z-axis motor 7021, an optical distance sensor 7022, a camera 7023, a 96-channel pick head unit 7024, and an integrated gas path on-off valve group 7025. The basic flow of monoclonal picking is as follows: firstly, the 96-channel monoclonal picking unit 702 is transferred to a target orifice plate position by the second X-Y linear motor gantry structure 701 to fulfill the initial aiming; then, the second X-Y linear motor gantry structure 701 drives the 96-channel monoclonal picking unit 702 to enable the optical distance sensor 7022 to scan the surface flatness of the picked plate and calibrate the height distance thereof; the camera 7023 captures images in real time and calculates the real-time position of the target bacteria or cells, and the control computer 10 sets the picking sequence schedules; then, the position of the target bacteria or cells is selected, and the second X-Y linear motor gantry structure 701 moves to the precise aiming position; the orifice plate image capturing assembly 703 quickly confirms the position, and fine adjustment is performed using the Z-axis motor 7021 to move the pick head to a local height matching the picking position; a single-channel gas valve in the integrated gas path on-off valve group 7025 is actuated to fulfill up-and-down movement of the single-channel pick head to thereby carry out the picking operation. A next target is chosen for monoclonal picking by the same step as performed on the previous target until all the monoclonal picking products on the whole 96-well plate have been completely eluted. The pick heads in the 96-channel pick head unit 7024 have tip ends specially shaped to reach the best picking effect and transfer safety, and to be prevented from falling during transfer. The pick heads are made of SUS316 material that meets GMP requirements to keep the sample clean. The guide portion of the pick head is made of high wear-resistant materials, which meet FDA certification to avoid dust and particulate contamination during movement. The floating compensation and connection arrangement between the pick head and the driving mechanism reduces the processing and assembling difficulty while ensuring the relative accuracy of the multi-channel pick heads. The drive unit and the control element of the pick heads employ modular design, which can flexibly realize arbitrary throughput integration from single channel to 96 channels. The tip ends of the multi-channel pick heads are hard limited, and a flat surface formed in a single processing step ensures the accuracy of the descending distance of the pick heads. The mounting hole of the multi-channel pick heads is formed in a single processing step to ensure the accuracy of the relative position of the multi-channel pick heads. The multi-channel drive part fixing mechanism adopts modular design, which can flexibly realize arbitrary throughput integration from single channel to 96 channels. The pick head sterilizing and cleaning assembly 704 includes a pick head alcohol-cleaning tank 1# 7041, a pick head alcohol-cleaning tank 2# 7042, a pick head alcohol-cleaning tank 3# 7043, a pick head drying tank 7044, an air inlet 1# 7045, an air inlet 2# 7046, a heating unit array 7047 and an air outlet 7048. The basic flow of cleaning the pick head is as follows: the pick head is sent to the pick head alcohol-cleaning tank 1# 7041, the pick head alcohol-cleaning tank 2# 7042 and the pick head alcohol-cleaning tank 3# 7043 in order for cycling alcohol-cleaning; the heating unit array 7047 dries the pick head by heat source irradiation; and air flows in through the air inlet 1# 7045 and the air inlet 2# 7046 and is discharged through the air outlet 7048, whereby the flowing air may cool the pick head. The oscillation-coating assembly 709 includes a clamping structure 7091, a cam oscillating structure 7092, a drive motor 7093 and an in-situ sensor 7094. The basic process flow of oscillation coating is as follows: an agar plate to be coated is transferred into the oscillation-coating assembly 709; the multi-channel pipetting unit 705 fills the process liquid into each well of the agar plate; the drive motor 7093 drives the agar plate to oscillate through the cam oscillating structure 7092 based on the preset rotational speed, so as to realize uniform coating of the process liquid on the surface of the agar plate; the multi-channel pipetting unit 705 adds bacteria or cell solution to the surface of the agar plate; the drive motor 7093 drives the agar plate to oscillate through the cam oscillating structure 7092 based on the preset rotational speed, so as to realize uniform coating of the bacteria or cell solution on the surface of the agar plate.

Referring to Figs. 25-27, the workflow editing unit 12 has the functions of editing and managing the full-flow process list, providing a standardized process template, and controlling and adjusting the process in real time; the full-automatic device monitoring and operating unit 13 has the functions of managing the historical data of sample liquid operation, the historical data of sample magnetic beads operation, the historical data of sample coating operation, the historical data of sample monoclonal picking and the historical data of sample transferring operation, managing the state of materials at each station, monitoring the running state of modules of each device, managing the loading of solid consumables, managing the loading of liquid materials, managing the storage of consumables at the storage center, and managing the replenishment of consumables at the storage center; the manual device debugging unit 14 has the functions of calibrating and correcting each mobile module in the system, calibrating and correcting each liquid unit in the system, demonstrating the intelligent monoclonal sample picking, and demonstrating the sample coating process; the data storage unit 15 has the functions of storing original image data of monoclonal picking; the log prompting unit 16 has the functions of recording the operator's login information, material loading information, device failure information during operation, device warning information during operation, and device operation information during operation; and the parameter setting unit 17 has the functions of setting personnel's authority and parameters of each module in the system, configuring the types of liquid pipes, configuring the capacity of liquid, configuring the operation parameters of liquid units, configuring the intelligent image recognition of monoclonal samples, and configuring the coating movement parameters.

Although the exemplary embodiments of the present disclosure have been described above with reference to the accompanying drawings, those skilled in the art should understand that the present disclosure is not limited to the specific structure that has been disclosed. Multiple changes and modifications may be made to the exemplary embodiments without substantively departing from the spirit and scope of the present invention. Accordingly, all the changes and modifications are encompassed within the protection scope as defined by the claims of the present invention.

## Claims

1. An intelligent system for gene synthesis and monoclonal picking, comprising:
a core operation module for performing operations associated with gene synthesis and monoclonal picking, the core operation module at least including:
a liquid operation compartment for performing operations of mixing gene fragments and operations of mixing synthetizing reagents with biological samples in the process of gene synthesis;
a purification compartment for performing purification operations on nucleic acid products created after the mixing;
an amplification compartment for performing amplification operations and error correction operations on fragments of the nucleic acid products and performing culturing and amplification operations on vectors; and
a monoclonal picking compartment for performing monoclonal picking operations on purified and cultured nucleic acid products; and
a control system for controlling at least a part of operations of corresponding compartments of the core operation module.

2. The intelligent system for gene synthesis and monoclonal picking according to claim 1, wherein the core operation module further comprises a loading and unloading compartment for replenishing various materials required in the process of gene synthesis and monoclonal picking.

3. The intelligent system for gene synthesis and monoclonal picking according to claim 2, wherein the core operation module further comprises a transfer compartment for transferring biological samples and/or materials among at least a part of the liquid operation compartment, the purification compartment, the amplification compartment, the monoclonal picking compartment and the loading and unloading compartment.

4. The intelligent system for gene synthesis and monoclonal picking according to claim 3, wherein the transfer compartment includes a transfer robot.

5. The intelligent system for gene synthesis and monoclonal picking according to claim 3, wherein the transfer compartment is arranged at a middle position of the core operation module, while the liquid operation compartment, the purification compartment, the amplification compartment, the monoclonal picking compartment and the loading and unloading compartment are divided into two groups and arranged at both sides of the transfer compartment.

6. The intelligent system for gene synthesis and monoclonal picking according to claim 3, wherein the transfer compartment is arranged at a central position of the core operation module, while the liquid operation compartment, the purification compartment, the amplification compartment, the monoclonal picking compartment and the loading and unloading compartment are arranged around the transfer compartment along a circumference.

7. The intelligent system for gene synthesis and monoclonal picking according to claim 3, wherein at least a part of the liquid operation compartment, the purification compartment, the transfer compartment, the amplification compartment, the monoclonal picking compartment and the loading and unloading compartment are arranged in a compartment body capable of being closed tightly, the compartment body including a laminar flow unit to put corresponding compartments in a controlled laminar flow environment.

8. The intelligent system for gene synthesis and monoclonal picking according to claim 7, wherein the laminar flow unit comprises a laminar air-supply unit including an air-supply fan or at least one filtering unit, and a laminar air-discharge unit including an air-discharge fan and a reverse filtering unit.

9. The intelligent system for gene synthesis and monoclonal picking according to claim 7, wherein an ultraviolet irradiation unit is provided in the compartment body.

10. The intelligent system for gene synthesis and monoclonal picking according to claim 1, wherein the liquid operation compartment comprises at least one pipetting unit, the pipetting unit is mounted on a linear motor gantry main frame structure so as enable the pipetting unit to be movable along an X-axis; and wherein each pipetting unit comprises one or more pipetting heads each of which is independently movable along a Y-axis and a Z-axis in the pipetting unit.

11. The intelligent system for gene synthesis and monoclonal picking according to claim 10, wherein each pipetting unit includes one or more movers mounted along a direction of the Y-axis to enable each pipetting head in each pipetting unit to be movable along the Y-axis under driving of the movers; and wherein each pipetting unit further comprises a Z-axis driving mechanism to drive each pipetting head in each pipetting unit to move along the Z-axis.

12. The intelligent system for gene synthesis and monoclonal picking according to claim 10, wherein the liquid operation compartment further comprises one or more of a liquid pumping unit, a pump set array, a bottom-compartment liquid storage unit, a consumable stage sliding table array, an oscillation unit, a consumable carrier and an operation stage.

13. The intelligent system for gene synthesis and monoclonal picking according to claim 10, wherein the liquid operation compartment includes two or more pipetting units, which are configured to be able to perform operations independently of each other as well as to perform operations in parallel.

14. The intelligent system for gene synthesis and monoclonal picking according to claim 1, wherein the purification compartment comprises at least one purification pipetting unit, the at least one purification pipetting unit is mounted on a linear motor gantry main frame structure, so as to enable the at least one purification pipetting unit to be movable along an X-axis; and wherein each purification pipetting unit comprises one or more purification pipetting heads each of which is independently movable along a Y-axis and a Z-axis in the purification pipetting unit.

15. The intelligent system for gene synthesis and monoclonal picking according to claim 14, wherein the purification compartment further comprises one or more of a liquid pumping unit, a consumable stage sliding table array, a bottom-compartment liquid storage unit, an oscillation unit, a consumable carrier, an operation stage and a temperature-controlled storage device.

16. The intelligent system for gene synthesis and monoclonal picking according to claim 1, wherein the monoclonal picking compartment comprises a monoclonal picking unit for picking monoclonal nucleic acid products from an orifice plate.

17. The intelligent system for gene synthesis and monoclonal picking according to claim 16, wherein the monoclonal picking unit comprises a multi-channel pick head assembly formed by integrating a plurality of pick head elements.

18. The intelligent system for gene synthesis and monoclonal picking according to claim 17, wherein the multi-channel pick head assembly is connected to an integrated gas path on-off valve group, which is capable of controlling each pick head element separately to enable each pick head element to independently move and to independently fulfill operations of picking the monoclonal nucleic acid products.

19. The intelligent system for gene synthesis and monoclonal picking according to claim 16, wherein the monoclonal picking unit is mounted on an X-Y linear motor gantry main frame structure, so as to enable the monoclonal picking unit to be movable along an X-axis and a Y-axis, and the monoclonal picking unit includes a Z-axis motor, so as to enable the monoclonal picking unit to be movable along a Z-axis.

20. The intelligent system for gene synthesis and monoclonal picking according to claim 17, wherein the monoclonal picking unit further comprises an image capturing assembly for identifying and localizing target nucleic acid products in the monoclonal picking process.

21. The intelligent system for gene synthesis and monoclonal picking according to claim 20, wherein the image capturing assembly includes: a camera for real-time determining positions of nucleic acid products to be picked; and an optical distance sensor for calibrating a vertical distance between tip ends of the pick head elements in the multi-channel pick head assembly and a surface of the orifice plate bearing the nucleic acid products to be picked, and for evaluating a surface flatness of the orifice plate.

22. The intelligent system for gene synthesis and monoclonal picking according to claim 21, wherein the monoclonal picking unit further comprises a bracket assembly including vertical support plates and a plurality of horizontal support plates extending perpendicular to the vertical support plates, and wherein each horizontal support plate is provided with holes through which the plurality of pick head elements extend, so as to ensure accurate localization of the plurality of pick head elements.

23. The intelligent system for gene synthesis and monoclonal picking according to claim 22, wherein one of the plurality of horizontal support plates is also provided with a guiding and positioning mechanism for the plurality of pick head elements, so as to localize and guide vertical movement of the plurality of pick head elements in the multi-channel pick head assembly.

24. The intelligent system for gene synthesis and monoclonal picking according to claim 22, wherein an upper surface of a bottom horizontal support plate of the plurality of horizontal support plates is configured to function as a limiting surface for each pick head element in the multi-channel pick head assembly, in order to ensure that the tip ends of the plurality of pick head elements are at a same height.

25. The intelligent system for gene synthesis and monoclonal picking according to claim 24, wherein the optical distance sensor and/or the camera are also arranged on the upper surface of the bottom horizontal support plate.

26. The intelligent system for gene synthesis and monoclonal picking according to claim 17, wherein each pick head element includes a first section and a second section, both of which are configured to be in floating connection.

27. The intelligent system for gene synthesis and monoclonal picking according to claim 26, wherein each pick head element includes a floating connection mechanism which includes a sleeve element and a floating element, the floating element is configured to be mountable in the sleeve element and capable of floating a distance within the sleeve element.

28. The intelligent system for gene synthesis and monoclonal picking according to claim 27, wherein an upper end of the first section of each pick head element is connected with a drive control valve to drive vertical movement of the pick head element by the drive control valve, and a lower end of the first section of each pick head element is secured within the sleeve element of the floating connection mechanism.

29. The intelligent system for gene synthesis and monoclonal picking according to claim 28, wherein the lower end of the first section of each pick head element includes a threaded joint for threadedly connecting the lower end of the first section to the sleeve element of the floating connection mechanism.

30. The intelligent system for gene synthesis and monoclonal picking according to claim 17, wherein each pick head element further includes a guide element.

31. The intelligent system for gene synthesis and monoclonal picking according to claim 26, wherein each pick head element further comprises a guide element arranged on an outer periphery of the second section of the pick head element.

32. The intelligent system for gene synthesis and monoclonal picking according to claim 30 or 31, wherein the guide element is configured as a guide bearing.

33. The intelligent system for gene synthesis and monoclonal picking according to claim 17, wherein each pick head element is made of SUS316 stainless steel material.

34. The intelligent system for gene synthesis and monoclonal picking according to claim 16, wherein the monoclonal picking compartment further comprises one or more of an oscillation-coating unit, a multi-channel pipetting unit, a liquid pumping unit, a bottom-compartment liquid storage unit, a consumable stage sliding table array, an operation stage and a temperature-controlled storage device.

35. The intelligent system for gene synthesis and monoclonal picking according to claim 16, wherein the monoclonal picking compartment further comprises an oscillation-coating unit for realizing uniform distribution of flora on the orifice plate, and wherein the oscillation-coating unit comprises a clamping mechanism and a cam oscillating mechanism, the orifice plate capable of being clamped in the clamping mechanism and oscillated together with the clamping mechanism under the drive of the cam oscillating mechanism.

36. The intelligent system for gene synthesis and monoclonal picking according to claim 17, wherein the monoclonal picking compartment further comprises a pick head cleaning assembly including at least one pick head cleaning element, for cleaning the multi-channel pick head assembly of the monoclonal picking unit.

37. The intelligent system for gene synthesis and monoclonal picking according to claim 36, wherein the pick head cleaning assembly further comprises a pick head drying unit including a heating unit, for drying the pick head elements in the multi-channel pick head assembly of the monoclonal picking unit.

38. The intelligent system for gene synthesis and monoclonal picking according to claim 37, wherein the pick head drying unit includes an air-supply duct and an air-discharge duct, for cooling the pick head elements in the multi-channel pick head assembly of the monoclonal picking unit that have been dried.

39. The intelligent system for gene synthesis and monoclonal picking according to claim 1, wherein the control system includes a control computer, an I/O data collecting module, and a drive module.

40. The intelligent system for gene synthesis and monoclonal picking according to claim 39, wherein the control system comprises a software system, which includes one or more of a workflow editing unit, a device monitoring and operating unit, a device debugging unit, a data storage unit, a log prompting unit and a parameter setting unit.

41. The intelligent system for gene synthesis and monoclonal picking according to claim 1, wherein the intelligent system for gene synthesis and monoclonal picking further comprises at least one of the following modules: a sequencer module for detecting, confirming and screening synthesized gene fragments in the process of gene synthesis and monoclonal picking; and a material storage module for storing various materials required in the process of gene synthesis and monoclonal picking.
